(19) Europäisches Patentamt
European Patent Office
Office européen des brevets

(11) Publication number: **0 185 079**
**B1**

(12) # EUROPEAN PATENT SPECIFICATION

(45) Date of publication of patent specification: **19.10.88**

(21) Application number: **85903238.5**

(22) Date of filing: **01.06.85**

(86) International application number: **PCT/EP 85/00261**

(87) International publication number: **WO/8600066 (03.01.86 Gazette 86/01)**

(51) Int. Cl.[4]: **C 07 C103/84**, C 07 C103/76, C 07 C121/43, C 07 H 13/04, A 61 K 31/19, A 61 K 31/215, A 61 K 31/33, A 61 K 31/56, A 61 K 31/70

(54) **N-SUBSTITUTED BUTYRAMIDE DERIVATIVES.**

(30) Priority: **08.06.84 US 618617**

(43) Date of publication of application: **25.06.86 Bulletin 86/26**

(45) Publication of the grant of the patent: **19.10.88 Bulletin 88/42**

(84) Designated contracting states: **AT BE CH DE FR GB IT LI LU NL SE.**

(56) References cited:
**EP-A- 0 038 046**
**EP-A- 0 082 088**
**FR-A- 2 372 803**
**Chemische Berichte, volume 115, nr. 6, 1982, Weinheim, (DE) R.W. Hoffmann et al.: Stereoselektive Synthese von Alkoholen XI", pages 2357-2370, see page 2361**

(73) Proprietor: **CIBA-GEIGY AG**
**Klybeckstrasse 141**
**CH-4002 Basel (CH)**

(72) Inventor: **KSANDER, Gary, M.**
**RD 10 Ressique Street**
**Carmel, NY 10512 (US)**

**EP 0 185 079 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

## Description

EP-A-38046 discloses certain N-succinoyl derivates of α-amino acids with enkephalinase inhibitory activity. In EP-A-82088, α-amino acid derivatives inhibiting the enzyme enkephalinase are disclosed.

The present invention concerns new butyramide derivatives corresponding to formula I

$$\underset{R}{Y-CH}-CH_2-\underset{R_o}{CH}-CONH-A-X \qquad (I)$$

wherein X and Y independently represent hydroxymethyl ; cyano ; carboxy ; functionally modified carboxy selected from esterified carboxy, carbamoyl, and N-substituted carbamoyl ; 5-tetrazolyl ; 2-oxazolyl, 4,5-dihydro-2-oxazolyl, 2-imidazolyl or 4,5-dihydro-2-imidazolyl or any said grouping substituted by lower alkyl ; R and $R_0$ independently represent lower alkyl, ($C_3$-$C_7$)-cycloalkyl-lower alkyl, or aryl-lower alkyl in which aryl represents phenyl, pyridyl, thienyl, furyl, biphenylyl or naphthyl, each unsubstituted or mono- or di-substituted by halogen, lower alkyl, hydroxy, acyloxy, lower alkoxy, trifluoromethyl or cyano ; A represents straight chain ($C_2$-$C_5$)-alkylene ; or A represents straight chain ($C_2$-$C_5$)-alkylene substituted by lower alkyl, by lower alkylthio-lower alkyl, by hydroxy-lower alkyl, by acyloxy-lower alkyl, by lower alkoxy-lower alkyl, by amino or acylamino, by amino-lower alkyl, by acylamino-lower alkyl, by ($C_3$-$C_7$)-cycloalkyl, by ($C_3$-$C_7$)-cycloalkyl-lower alkyl, by aryl or aryl-lower alkyl in which aryl represents phenyl or phenyl mono- or disubstituted by halogen, lower alkyl, lower alkoxy, hydroxy, acyloxy, trifluoromethyl or cyano ; or A represents phenylene or cyclohexylene ; or pharmaceutically acceptable prodrug derivatives of any said compounds having a free carboxy group ; or pharmaceutically acceptable salts of any said compounds with a salt-forming group ; processes for the manufacture of these compounds ; pharmaceutical compositions comprising said compounds ; and their use as pharmaceutical agents or for the manufacture of pharmaceutical preparations.

Compounds of formula I, depending on the nature of R, $R_0$, X, Y and A possess a number of asymmetric carbon atoms. The resulting diastereoisomers and optical antipodes are encompassed by the instant invention.

The general definitions used herein unless denoted otherwise have the following meanings within the scope of the present invention.

Aryl represents a carbocyclic or heterocyclic aromatic radical preferably being phenyl, 2- or 3-thienyl, o-, m- or p-biphenylyl, 2- or 3-indolyl, 2-, 3- or 4-pyridyl, 1- or 2-naphthyl, 2- or 3-furyl, each optionally substituted by lower alkyl, lower alkoxy, lower alkylenedioxy, hydroxy, lower alkanoyloxy, halogen or trifluoromethyl.

Optionally substituted phenyl represents preferably phenyl or phenyl substituted by one to three of lower alkyl, lower alkoxy, halogen or trifluoromethyl.

Aryl, as in aryl-lower alkyl, is preferably phenyl or phenyl substituted by one to three of lower alkyl, lower alkoxy, hydroxy, halogen or trifluoromethyl ; and aryl-lower alkyl is advantageously benzyl or phenethyl optionally substituted by one to three of lower alkyl, lower alkoxy, hydroxy, lower alkanoyloxy, halogen or trifluoromethyl.

($C_1$-$C_{20}$)-Alkyl represents e.g. dodecyl, hexadecyl or 2,7-dimethyloctyl, and is preferably lower alkyl.

Phenylene represents o-, m- or p-phenylene.

Cyclohexylene represents 1,2-, 1,3- and preferably, 1,4-cyclohexylene.

Straight chain ($C_2$-$C_5$)-alkylene represents preferably ethylene or propylene.

The term cycloalkyl represents a cyclic hydrocarbon radical which contains 3 to 7 ring carbons and is, for example, cyclopentyl or cyclohexyl.

The term cycloalkyl-lower alkyl represents preferably 1- or 2-(cyclopentyl or cyclohexyl) ethyl, 1-, 2- or 3-(cyclopentyl or cyclohexyl) propyl, or 1-, 2-, 3- or 4-(cyclopentyl or cyclohexyl)butyl.

The term « lower » referred to above and hereinafter in connection with organic radicals or compounds respectively defines for example such with up to and including 7, preferably up and including 4 and advantageously one or two carbon atoms. A lower alkyl group preferably contains 1-4 carbon atoms and represents for example ethyl, propyl, butyl or advantageously methyl.

A lower alkoxy group preferably contains 1-4 carbon atoms and represents for example methoxy, propoxy, isopropoxy or advantageously ethoxy.

A mono-lower alkylamino group preferably contains 1-4 carbon atoms in the alkyl portion and is for example N-methylamino, N-propylamino or advantageously N-ethylamino.

A di-lower alkylamino group preferably contains 1-4 carbon atoms in each lower alkyl portion and represents, for example N,N-dimethylamino, N-methyl-N-ethylamino and advantageously N,N-diethylamino. A lower alkoxycarbonyl group preferably contains 1 to 4 carbon atoms in the alkoxy portion and represents, for example, methoxycarbonyl, propoxycarbonyl, isopropoxycarbonyl or advantageously ethoxycarbonyl.

Lower alkylenedioxy represents preferably ethylenedioxy, and advantageously methylenedioxy.

Aryl-lower alkoxy represents advantageously e.g. benzyloxy, benzyloxy substituted by methyl,

methoxy or chloro, and pyridylmethoxy. Pyridyl represents 2-, 3- or 4-pyridyl.

Carboxy-lower alkoxy represents advantageously e.g. 1-carboxyethoxy. Lower alkoxycarbonyl-lower alkoxy represents advantageously e.g. 1-(ethoxycarbonyl) ethoxy.

Amino-lower alkoxy, mono-lower alkylamino-lower alkoxy, di-lower alkylamino-lower alkoxy advantageously represent respectively e.g. aminoethoxy, ethylaminoethoxy, diethylaminoethoxy, and especially ω-(amino, ethylamino, diethylamino)-ethoxy respectively.

Hydroxy-lower alkyl is preferably hydroxymethyl, hydroxyethyl or hydroxypropyl, advantageously hydroxymethyl.

Bicycloalkyloxycarbonyl-lower alkoxy preferably represents bicyclo-[2,2,1] heptyloxycarbonyl-lower alkoxy unsubstituted or substituted by lower alkyl, advantageously bornyloxycarbonylmethoxy.

Amino-lower alkyl represents preferably amino-(ethyl, propyl or butyl).

Lower alkylidene is preferably isopropylidene. Cycloalkylidene is preferably cyclohexylidene.

Halogen preferably represents chlorine, but may also be bromine, fluorine or iodine.

Lower alkanoyl is preferably acetyl, propionyl, butyryl, or pivaloyl.

Acyl in acyloxy, acyloxy-lower alkyl, acylamino, acylamino-lower alkyl represents lower alkanoyl, aroyl, lower alkoxycarbonyl, or di-lower alkylcarbamoyl, preferably lower alkanoyl.

Aroyl is preferably benzoyl or benzenesulfonyl ; benzoyl or benzenesulfonyl substituted by one to three of lower alkyl, lower alkoxy, halogen or trifluoromethyl ; or heteroaroyl, e.g. thienoyl, pyrroloyl, 2-, 3- or 4-pyridylcarbonyl, advantageously nicotinoyl.

Lower alkanoyloxy is preferably acetoxy, pivaloyloxy or propionyloxy ; lower alkanoylamino is preferably acetylamino or propionylamino ; aroyloxy is preferably benzenesulfonyloxy, benzoyloxy, benzoyloxy or benzenesulfonyloxy substituted on the benzene ring by one to three of lower alkyl, lower alkoxy, halogen or trifluoromethyl, or heteroaroyloxy.

Heteroaroyloxy is preferably 2-, 3- or 4-pyridylcarbonyloxy, advantageously nicotinoyloxy.

Acylamino represents lower alkanoylamino, aroylamino, heretoaroylamino, lower alkoxycarbonylamino, or di-lower alkylcarbamoylamino, preferably lower alkanoylamino.

Acylamino-lower alkyl represents preferably acylamino-(ethyl, propyl or butyl).

Mono- or di-lower alkylcarbamoyl is preferably mono-N- or di-N,N-(methyl, ethyl, propyl)-carbamoyl.

Functionally modified carboxy represents esterified carboxy, carbamoyl or carbamoyl substituted on nitrogen.

Esterified carboxy represents preferably carboxy esterified in form of a pharmaceutically acceptable ester, advantageously an ester that may be convertible by solvolysis or under physiological conditions to the free carboxylic acid, e. g. especially optionally substituted alkoxycarbonyl in which optionally substituted lower alkoxy represents preferably ($C_1$-$C_{20}$)-alkoxy, advantageously lower alkoxy ; (amino, acylamino, mono- or di-lower alkylamino)-lower alkoxy ; carboxy-lower alkoxy, e. g. alpha-carboxy-lower alkoxy ; lower alkoxycarbonyl-lower alkoxy, e. g. alpha-lower alkoxycarbonyl-lower alkoxy ; aryl-lower alkoxy, preferably optionally (halogen, lower alkyl or lower alkoxy)-benzyloxy or pyridyl-methoxy ; (hydroxy, lower alkanoyloxy or lower alkoxy)-lower alkoxy, e. g. pivaloyloxymethoxy ; (hydroxy, lower alkanoyloxy or lower alkoxy)-lower alkoxymethoxy ; bicycloalkoxycarbonyl-lower alkoxy, e. g. bicyclo [2,2,1]-heptyloxycarbonyl-lower alkoxy, especially bicyclo-[2,2,1]-heptyloxycarbonylmethoxy such as bornyloxycarbonylmethoxy ; 1-(lower alkoxycarbonyloxy)-lower alkoxy. Esterified carboxy also represents a phthalidyl ester, such as 3-phthalidoxycarbonyl or (lower alkyl, lower alkoxy, halogen)-substituted 3-phthalidoxycarbonyl.

Esterified carboxy further represents :

(a) cholestan-3-oxycarbonyl or cholest-5-en-3-oxycarbonyl ;

(b) monosaccharidyloxycarbonyl or protected monosaccharidyloxycarbonyl in which monosaccharidyloxy and protected monosaccharidyloxy represent preferably glucosyloxy, galactosyloxy, mannosyloxy, sorbosyloxy, allosyloxy, ribosyloxy, arabinosyloxy, ribonyloxy, gluconyloxy, or cyclic, e. g. appropriate pyranose, furanose or lactone forms thereof, wherein hydroxy groups are free or one or more, as appropriate, are protected in form of esters, e. g. a lower alkanoyl or a benzoyl ester, in form of ethers, e. g. a lower alkyl or benzyl ether, or, in case two vicinal hydroxy groups are involved, in the form of acetals or ketals, e. g. a lower alkylidene, a benzylidene or a 5- or 6-membered cycloalkylidene derivative ;

(c) polyhydroxy-lower alkoxycarbonyl or protected polyhydroxy-lower alkoxycarbonyl in which polyhydroxy-lower alkoxy and protected polyhydroxy-lower alkoxy represent preferably dihydroxypropyloxy or trihydroxybutyloxy wherein hydroxy groups are free or one or more, as appropriate, are protected in form of esters, e. g., a lower alkanoyl or a benzoyl ester, in form of ethers, e. g. a lower alkyl or benzyl ether, or, in case two vicinal hydroxy groups are involved, in the form of acetals or ketals, e. g. a lower alkylidene, a benzylidene or a 5- or 6-membered cycloalkylidene derivative.

Protected monosaccharidyloxy represents preferably
1,2 : 5,6-di-O-isopropylidene-D-glucofuranos-3-yloxy,
1,2 : 3,4-di-O-isopropylidene-D-galactopyranos-6-yloxy,
2,3-O-isopropylidene-D-ribono-(1,4-lactone)-5-yloxy,
2,3 : 5,6-di-O-cyclohexylidene-D-mannofuranos-1-yloxy,
2,3-O-cyclohexylidene-D-ribono-(1,4-lactone)-5-yloxy,

1-methyl-2,3-O-isopropylidene-D-ribofuranos-5-yloxy,
1,2-O-isopropylidene-D-glucofuranos-3-yloxy,
2,3 : 4,6-di-O-isopropylidene-L-sorbofuranos-1-yloxy,
1,2 : 5,6-di-O-isopropylidene-D-allofuranos-3-yloxy,
2,3 : 5,6-di-O-isopropylidene-D-mannofuranos-1-yloxy,
2,3,5-tri-O-benzyl-D-arabofuranos-1-yloxy,
2,3,4,6-tetra-O-benzyl-D-glucopyranos-1-yloxy or
2,3-O-benzylidene-D-ribono-(1,4-lactone)-5-yloxy.

Protected polyhydroxy-lower alkoxy represents preferably (2,2-dimethyl-1,3-dioxolan-4-yl)-methoxy.

Optionally substituted carbamoyl represents optionally substituted aminocarbonyl in which optionally substituted amino represents preferably amino ; lower alkylamino ; di-lower alkylamino ; morpholino ; N-lower alkyl-piperazino ; pyrrolidino ; piperidino ; perhydroazepino ; (amino or acylamino)-lower alkylamino ; alpha-(carboxy or lower alkoxycarbonyl)-lower alkylamino ; aryl-lower alkylamino in which aryl is preferably phenyl or indolyl and which can be substituted on the alpha-carbon by carboxy or lower alkoxycarbonyl ; hydroxyamino ; or lower alkylsulfonylamino.

Pharmaceutically acceptable salts are acid addition salts, which are preferably such of therapeutically acceptable inorganic or organic acids, such as strong mineral acids, for example hydrohalic, e. g. hydrochloric or hydrobromic acid, sulfuric, phosphoric or nitric acid ; aliphatic or aromatic carboxylic or sulfonic acids, e. g. formic, acetic, propionic, succinic, glycollic, lactic, malic, tartaric, gluconic, citric, maleic, fumaric, pyruvic, phenylacetic, benzoic, 4-aminobenzoic, anthranilic, 4-hydroxybenzoic, salicylic, 4-aminosalicylic, pamoic, nicotinic, methanesulfonic, ethanesulfonic, hydroxyethanesulfonic, benzenesulfonic, p-toluenesulfonic, naphthalenesulfonic, sulfanilic or cyclohexylsulfamic acid ; or ascorbic acid.

The compounds of the invention exhibit valuable pharmacological properties, particularly potentiation of enkephalins by virtue of their ability to inhibit the enkephalin degrading enzyme enkephalinase. The compounds thus, for example, increase the level of endogenous enkephalins, e. g. met-enkephalin and leu-enkephalin, in mammals via inhibition of their degradation by the enzyme enkephalinase.

The foregoing attributes render the compounds of this invention particularly useful when administered, alone or in combination, to mammals e. g. for the treatment of conditions responsive to inhibition of enkephalinase, e. g. as analgesic, anticonvulsant, psychotropic (particularly antidepressant and neuroleptic), cardiovascular (particularly antihypertensive), as well as antiinflammatory agents. Furthermore, they exhibit only minimal side effects, especially minimal CNS side effects.

The above cited properties are demonstrable in vitro and in vivo tests, using advantageously mammals, e. g. mice, rats, dogs, monkeys or isolated organs, tissues and preparations thereof. Said compounds can be applied in vitro in the form of solutions, e. g. preferably aqueous solutions, and in vivo either enterally, parenterally, advantageously intravenously, or intracerebroventricularly, e. g. as a suspension or in aqueous solution. The dosage in vitro may range between about $10^{-4}$ molar and $10^{-9}$ molar concentrations. The dosage in vivo may range depending on the route of administration, between about 0.001 and 50 mg/kg, preferably between about 0.005 and 30 mg/kg, advantageously between about 0.01 and 20 mg/kg.

The enkephalinase inhibitory activity can be determined e. g. in vitro by an adaptation of the method of Alstein et al. as described in Life Sciences 28, 185 (1981), or in vivo, e. g. by measuring the potentiation of the analgesic activity of intracerebrally administered D-Ala$^2$-met$^5$-enkephalinamide or met enkephalin in mice. The enkephalinase inhibitory activity is also determined in vivo by measuring the increase in endogenous brain enkephalin levels in mice. The analgesic activity is demonstrable e. g. by potentiation of the analgesic effects of enkephalin and derivatives thereof, and by classical analgesic tests, such as the phenyl-p-benzoquinone induced writing test [J. Pharmacol. Exp. Therap. 125, 237 (1959)] and the hot plate test in the mouse [J. Pharmacol. Exp. Therap. 107, 385 (1953)]. The antihypertensive activity may be determined e. g. in the spontaneously hypertensive rat, Goldblatt rat or dog by direct measurement of systolic blood pressure.

More specifically, the instant invention relates to the glutaric acid derivatives of formula II

$$HOOC-\underset{R_1}{CH}-CH_2-\underset{R_2}{CH}-CONH-A-COOH \qquad (II)$$

wherein $R_1$ and $R_2$ independently represent lower alkyl, ($C_3$-$C_7$)-cycloalkyl-lower alkyl, or aryl-lower alkyl in which aryl represents phenyl, pyridyl, thienyl, furyl, biphenylyl or naphthyl each unsubstituted or mono- or di-substituted by halogen, lower alkyl, hydroxy, lower alkoxy, trifluoromethyl or cyano ; A represents straight chain ($C_2$-$C_5$)-alkylene ; or A represents straight chain ($C_2$-$C_5$)-alkylene substituted by lower alkyl, by lower alkylthio-lower alkyl, by hydroxy-lower alkyl, by acyloxy-lower alkyl, by lower alkoxy-lower alkyl, by amino or acylamino, by amino-lower alkyl, by acylamino-lower alkyl, by ($C_3$-$C_7$)-cycloalkyl, by ($C_3$-$C_7$)-cycloalkyl-lower alkyl, by aryl or aryl-lower alkyl in which aryl represents phenyl or phenyl mono- or disubstituted by halogen, lower alkyl, hydroxy, lower alkoxy, trifluoromethyl or cyano ; or A represents phenylene or cylohexylene ; a mono- or bis-carboxylic acid derivative thereof in which the derivative is

selected from an unsubstituted amide or mono- or di-($C_1$-$C_{20}$)-alkylamide ; a tertiary lower alkylene, oxalkylene or azaalkylene amide wherein the lower alkylene, oxalkylene or azaalkylene group together with the amide nitrogen forms a 5-, 6- or 7-membered ring, or said lower alkylene, oxalkylene or azaalkylene amide is substituted on the ring by lower alkyl, hydroxy-lower alkyl or by lower alkanoyloxy-lower alkyl ; an alpha-lower alkoxycarbonyl- or alpha-carboxy-substituted lower alkylamide ; an alpha-lower alkoxycarbonyl- or alpha-carboxy-substituted aryl-lower alkylamide in which aryl represents optionally substituted phenyl as defined above or 3-indolyl ; an (amino or acylamino)-lower alkylamide ; a ($C_1$-$C_{20}$)-alkyl ester ; an (amino, acylamino, mono- or di-lower alkylamino, carboxy or lower alkoxycarbonyl)-substituted lower alkyl ester ; an aryl-lower alkyl ester in which aryl represents optionally substituted phenyl as defined above or pyridyl ; a lower alkanoyloxy-lower alkyl ester ; a phthalidyl ester ; a (hydroxy, lower alkanoyloxy, or lower alkoxy)-substituted lower alkoxymethyl ester ; a bicycloalkyloxycarbonyl-lower alkyl ester having up to 10 carbon atoms in the bicycloalkyl group ; a hydroxyamide ; a lower alkylsulfonylamide ; or a 1-(lower alkoxycarbonyloxy)-lower alkyl ester ; a 3-cholestanyl or 3-cholestenyl ester ; and also a monosaccharidyl or protected monosaccharidyl ester being an ester incorporating as the alcohol portion a monosaccharide or protected monosaccharide, e. g. a free or protected aldopentose, aldohexose, ketopentose or ketohexose, in straight chain or cyclic form, e. g. furanose or pyranose form, or a free or protected glyconic acid of 5 or 6 carbon atoms or a lactone thereof ; a polyhydroxy-lower alkyl or protected polyhydroxy-lower alkyl ester being an ester incorporating as the alcohol portion a polyhydroxy-lower alkane or protected polyhydroxy-lower alkane, e. g. a free or protected glycerol or erythritol ; and pharmaceutically acceptable salts of any said compound with a salt-forming group.

More particularly, the instant invention relates to the compounds of formula III

$$R_3-\overset{O}{\overset{\|}{C}}-\underset{R_1'}{CH}-CH_2-\underset{R_2'}{CH}-CONH-A-\overset{O}{\overset{\|}{C}}-R_4 \qquad \text{(III)}$$

wherein $R_1'$ and $R_2'$ independently represent lower alkyl or aryl-($C_1$-$C_4$)-alkyl in which aryl represents phenyl or phenyl mono- or di-substituted by halogen, lower alkyl, hydroxy, lower alkoxy, trifluoromethyl or cyano ; A represents straight chain ($C_2$-$C_5$)-alkylene ; or A represents straight chain ($C_2$-$C_5$)-alkylene monosubstituted by lower alkyl, by phenyl or phenyl-lower alkyl, by (halogen, lower alkyl, hydroxy, trifluoromethyl or lower alkoxy)-mono- or di-substituted phenyl or phenyl-lower alkyl, by lower alkylthio-lower alkyl, by hydroxy-lower alkyl, by lower alkoxy-lower alkyl, by amino-lower alkyl or by acylamino-lower alkyl ; or A represents phenylene or cyclohexylene ; in which $R_3$ and $R_4$ independently represent hydroxy, ($C_1$-$C_{20}$)-alkoxy ; (amino, acylamino, mono- or di-lower alkylamino)-lower alkoxy ; carboxy-lower alkoxy ; lower alkoxycarbonyl-lower alkoxy ; aryl-lower alkoxy in which aryl represents optionally (halogen, lower alkyl, hydroxy or lower alkoxy)-mono- or di-substituted phenyl or pyridyl ; (hydroxy, lower alkanoyloxy or lower alkoxy)-lower alkoxy ; (hydroxy, lower alkanoyloxy or lower alkoxy)-lower alkoxymethoxy ; bicyclo [2,2,1]-heptyloxycarbonyl-lower alkoxy ; cholestan-3-oxy or cholest-5-en-oxy ; 3-phthalidoxy ; furthermore, (lower alkyl, lower alkoxy, halogen)-substituted 3-phthalidoxy, also monosaccharidyloxy or protected monosaccharidyloxy representing e. g. glucosyloxy, galactosyloxy, mannosyloxy, sorbosyloxy, allosyloxy, ribosyloxy, arabinosyloxy, ribonyloxy, gluconyloxy, or cyclic, e. g. appropriate pyranose, furanose or lactone forms thereof, wherein hydroxy groups are free or one or more, as appropriate, are protected in form of esters, e. g., a lower alkanoyl or a benzoyl ester, in form of ethers, e. g. a lower alkyl or benzyl ether, or, in case two vicinal hydroxy groups are involved, in the form of acetals or ketals, e. g. a lower alkylidene, a benzylidene or a 5- or 6-membered cycloalkylidene derivative ; and also polyhydroxy-lower alkoxy or protected polyhydroxy-lower alkoxy representing, e. g., dihydroxypropyloxy or trihydroxybutyloxy wherein hydroxy groups are free or one or more, as appropriate, are protected in form of esters, e. g., a lower alkanoyl or a benzoyl ester, in form of ethers, e. g. a lower alkyl or benzyl ether, or, in case two vicinal hydroxy groups are involved, in the form of acetals or ketals, e. g. a lower alkylidene, a benzylidene or a 5- or 6-membered cycloalkylidene derivative ; and also 1-(lower alkoxycarbonyloxy)-lower alkoxy ; amino ; mono- or di-($C_1$-$C_{20}$)-alkylamino ; morpholino ; N-lower alkylpiperazino ; pyrrolidino ; piperidino ; perhydroazepino ; (amino or acylamino)-lower alkylamino ; alpha-(carboxy or lower alkoxycarbonyl)-lower alkylamino ; aryl-lower alkylamino in which aryl is phenyl or 3-indolyl and which can be substituted on the alpha-carbon by carboxy or lower alkoxycarbonyl ; hydroxyamino ; or lower alkylsulfonylamino ; and pharmaceutically acceptable salts of any said compounds with a salt forming group.

Any pharmaceutically acceptable prodrug derivatives, e. g. any pharmaceutically acceptable mono- or di-(esters or amides), inclusive monoester-monoamide derivatives, of the di-carboxylic acids of this invention that may be convertible by solvolysis or under physiological conditions to the carboxylic acids e. g. esters and amides cited above, represent a particular object of the invention. Preferred as pharmaceutically acceptable prodrug derivatives are the pharmaceutically acceptable mono- or di-esters, especially the mono-esters of said carboxylic acids as defined herein.

Said esters are preferably, e. g., the alkyl, the pivaloyloxymethyl, bornyloxycarbonylmethyl, benzyl, pyridylmethyl, alpha-carboxyethyl, esterified alpha-carboxyethyl, (N-lower alkyl- or N-benzyl-1,4-dihyd-

ropyridyl-3-carbonyl)-amino-($C_2$-$C_5$)-alkyl, 1-(lower alkoxycarbonyloxy)-lower alkyl esters, the cholestan-3-yl or cholest-5-en-3-yl esters ; furthermore, 3-phthalidyl or (lower alkyl, lower alkoxy, halogen)-substituted 3-phthalidyl esters, the monosaccharidyl or protected monosaccharidyl esters being esters incorporating as the alcohol portion a monosaccharide or protected monosaccharide, e. g. a free or protected aldopentose, aldohexose, ketopentose or ketohexose, in straight chain or cyclic form, e. g., furanose or pyranose form ; or a free or protected glyconic acid of 5 or 6 carbon atoms or a lactone thereof ; the polyhydroxy-lower alkyl or protected polyhydroxy-lower alkyl esters being esters incorporating as the alcohol portion a polyhydroxy-lower alkane or protected polyhydroxy-lower alkane, e. g. a free or protected glycerol or erythritol.

Said amides are preferably e. g. simple primary and secondary amides, such as the unsubstituted amide or mono-($C_1$-$C_{20}$)-alkylamide, and amides derived from alpha-amino acids or derivatives thereof, such as the amides derived from alanine or phenylalanine.

Preferred are the compounds of formula III wherein $R_1'$ and $R_2'$ independently represent aryl-($C_1$-$C_4$)-alkyl in which aryl represents phenyl or phenyl mono- or di-substituted by halogen, lower alkyl, hydroxy, lower alkoxy, trifluoromethyl or cyano; A represents straight chain ($C_2$-$C_5$)-alkylene, straight chain ($C_2$-$C_5$)-alkylene substituted by lower alkyl, phenyl, halophenyl, or substituted by phenyl-lower alkyl ; or A represents phenylene or cyclohexylene ; $R_3$ and $R_4$ independently represent hydroxy ; ($C_1$-$C_{20}$)-alkoxy ; (amino, mono- or di-lower alkylamino)-lower alkoxy ; alpha-carboxy-lower alkoxy ; alpha-lower alkoxycarbonyl-lower alkoxy ; aryl-methoxy in which aryl represents phenyl, pyridyl, or (halogen, lower alkyl or lower alkoxy)-monosubstituted phenyl or pyridyl ; (lower alkanoyloxy or lower alkoxy)-methoxy ; (hydroxy, lower alkanoyloxy or lower alkoxy)-ethoxymethoxy ; bicyclo [2,2,1] heptyloxycarbonylmethoxy ; 1-(lower alkoxycarbonyloxy)-lower alkoxy ; furthermore, 3-phthalidoxy or (lower alkyl, lower alkoxy, halogen)-substituted 3-phthalidoxy, cholestan-3-oxy or cholest-5-en-3-oxy ; also monosaccharidyloxy or protected monosaccharidyloxy selected from glucosyloxy, galactosyloxy, mannosyloxy, sorbosyloxy, allosyloxy, ribosyloxy, arabinosyloxy, ribonyloxy, gluconyloxy, or cyclic, e. g. appropriate pyranose, furanose or lactone forms thereof, wherein hydroxy groups are free or one or more, as appropriate, are protected in form of a lower alkanoyl or a benzoyl ester, in form of a lower alkyl or benzyl ether, or, in case two vicinal hydroxy groups are involved, in the form of a lower alkylidene, a benzylidene or a 5- or 6-membered cycloalkylidene derivative ; polyhydroxy-lower alkoxy or protected polyhydroxy-lower alkoxy selected from dihydroxypropyloxy or trihydroxybutyloxy wherein hydroxy groups are free or one or more, as appropriate, are protected in form of a lower alkanoyl or a benzoyl ester, in form of a lower alkyl or benzyl ether, or, in case two vicinal hydroxy groups are involved, in the form of a lower alkylidene, a benzylidene or a 5- or 6-membered cycloalkylidene derivative ; and also amino ; mono- or di-($C_1$-$C_{20}$)-alkylamino ; morpholino ; N-methylpiperazino ; piperidino ; perhydroazepino ; amino-lower alkylamino ; alpha-(carboxy or lower alkoxycarbonyl)-lower alkylamino ; phenyl-alpha-(carboxy or lower alkoxycarbonyl)-lower alkylamino ; hydroxyamino ; or lower alkylsulfonylamino ; and pharmaceutically acceptable salts of any said compounds with a basic or acidic salt-forming group.

Further preferred are the above compounds of formula III wherein $R_3$ and $R_4$ independently represent hydroxy, ($C_1$-$C_{20}$)-alkoxy, pivaloyloxymethoxy, bornyloxycarbonylmethoxy, benzyloxy, pyridylmethoxy, alpha-carboxyethoxy, alpha-lower alkoxycarbonylethoxy, 3-phthalidoxy, furthermore monosaccharidyloxy or protected monosaccharidyloxy selected from glucofuranosyloxy, glucopyranosyloxy, galactopyranosyloxy, allofuranosyloxy, mannofuranosyloxy, ribofuranosyloxy, sorbofuranosyloxy, arabinofuranosyloxy and ribono-(1,4-lactone)-yloxy, wherein hydroxy groups are free or hydroxy groups are protected in form of a lower alkanoyl ester, in form of a benzyl ether or in form of an isopropylidene, a benzylidene or cyclohexylidene derivative ; and also amino, mono- or di-lower alkylamino, morpholino, alpha-(carboxy or lower alkoxycarbonyl)-lower alkylamino, or lower alkylsulfonylamino ; and pharmaceutically acceptable salts of any said compounds with a basic or acid salt-forming group.

Further preferred are the compounds of formula III wherein $R_1'$, $R_2'$ and A have meaning as defined above ; $COR_3$ and $COR_4$ represent independently carboxy or carboxy esterified in form of a pharmaceutically acceptable ester wherein $R_3$ and $R_4$ independently represent hydroxy, lower alkoxy, benzyloxy, pyridylmethoxy, pivaloyloxymethoxy, 3-phthalidoxy, also monosaccharidyloxy or protected monosaccharidyloxy as defined above.

Particularly preferred are the above compounds of formula III wherein one of $R_3$ and $R_4$ represents hydroxy and the other of $R_3$ and $R_4$ represents protected monosaccharidyloxy as defined above. Also are particularly preferred the above compounds of formula III wherein one of $R_3$ and $R_4$ represents hydroxy and the other of $R_3$ and $R_4$ represents 3-phthalidoxy.

A specific embodiment of the invention is represented by the glutaric acid derivatives of formula IV

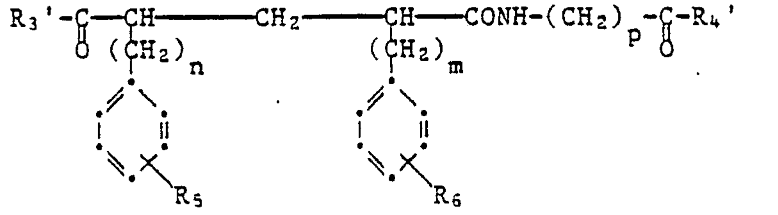

(IV)

wherein m and n independently represent an integer from 1 to 4 ; p represents an integer from 2 to 4 ; $R_3'$ and $R_4'$ represent hydroxy ; $R_5$ and $R_6$ independently represent hydrogen, halogen, hydroxy, lower alkoxy, lower alkyl or trifluoromethyl ; or a pharmaceutically acceptable mono- or di-(ester or amide) prodrug derivative thereof ; and pharmaceutically acceptable salts of any said compounds with a free carboxy group or basic salt-forming group.

A further embodiment of the invention is represented by the compounds of formula IV wherein m and n independently represent an integer from 1 to 4 ; p represents an integer from 2 to 4 ; $R_3'$ and $R_4'$ independently represent hydroxy, ($C_1$-$C_{20}$)-alkoxy, amino, pivaloyloxymethoxy, bornyloxycarbonylmethoxy, benzyloxy, pyridylmethoxy, 1-(lower alkoxycarbonyloxy)-lower alkoxy or lower alkylsulfonylamino, furthermore 3-phthalidoxy ; and also monosaccharidyloxy or protected monosaccharidyloxy selected from glucofuranosyloxy, glucopyranosyloxy, galactopyranosyloxy, allofuranosyloxy, mannofuranosyloxy, ribofuranosyloxy, sorbofuranosyloxy, arabinofuranosyloxy and ribono(1,4-lactone)-yloxy, wherein hydroxy groups are free or hydroxy groups are protected in form of a lower alkanoyl ester, in form of a benzyl ether or in form of an isopropylidene, a benzylidene or cyclohexylidene lower derivative ; $R_5$ and $R_6$ independently represent hydrogen, halogen, hydroxy, lower alkoxy, lower alkyl or trifluoromethyl ; and pharmaceutically acceptable salts of any said compounds with a free carboxy group or basic salt-forming group.

Further preferred are the compounds of formula IV wherein m and n independently represent the integer 1 or 2 ; p represents the integer 2 or 3 ; $R_3'$ and $R_4'$ independently represent hydroxy, lower alkoxy, pivaloyloxymethoxy, pyridylmethoxy, benzyloxy, amino or lower alkylsulfonylamino, furthermore 3-phthalidoxy ; and also monosaccharidyloxy or protected monosaccharidyloxy as defined above ; and $R_5$ and $R_6$ have meanings described above.

Most preferred are the compounds of formula IV wherein m and n represent the integer 1 ; p represents the integer 2 or 3 ; $R_5$ and $R_6$ represent hydrogen ; $R_3'$ and $R_4'$ represent hydroxy ; pharmaceutically acceptable mono- or di-(ester or amide) prodrug derivatives thereof as defined above ; and pharmaceutically acceptable salts thereof.

A di-(ester or amide) prodrug derivative of a compound of formula IV is also intended to comprise such compounds having one ester and one amide group.

Preferred as mono- or di-(ester or amide) prodrug derivatives of the compounds of formula IV wherein $R_3'$ and $R_4'$ represent hydroxy are the compounds of formula IV wherein either one or both of $R_3'$ and $R_4'$ represent independently lower alkoxy of 1 to 4 carbon atoms, 3-pyridylmethoxy, benzyloxy, pivaloyloxymethoxy, bornyloxycarbonylmethoxy, 1-(ethoxycarbonyloxy)-ethoxy or amino, and pharmaceutically acceptable salts thereof.

Also preferred as mono- or di-(ester or amide) prodrug derivatives of the compounds of formula IV wherein $R_3'$ and $R_4'$ represent hydroxy are the monosaccharidyl esters wherein one of $R_3'$ and $R_4'$ represents preferably 1,2 : 5,6-di-O-isopropylidene-D-glucofuranos-3-yloxy, 1,2 : 3,4-di-O-isopropylidene-D-galactopyranos-6-yloxy, 2,3-O-isopropylidene-D-ribono-(1,4-lactone)-5-yloxy, 2,3 : 5,6-di-O-cyclohexylidene-D-mannofuranos-1-yloxy, 2,3-O-cyclohexylidene-D-ribono-(1,4-lactone)-5-yloxy, 1-methyl-2,3-O-isopropylidene-D-ribofuranos-5-yloxy, 1,2-O-isopropylidene-D-glucofuranos-3-yloxy, 2,3 : 4,6-di-O-isopropylidene-L-sorbofuranos-1-yloxy, 1,2 : 5,6-di-O-isopropylidene-D-allofuranos-3-yloxy, 2,3 : 5,6-di-O-isopropylidene-D-mannofuranos-1-yloxy, 2,3,5-tri-O-benzyl-D-arabofuranos-1-yloxy, 2,3,4,6-tetra-O-benzyl-D-glucopyranos-1-yloxy or 2,3-O-benzylidene-D-ribono-(1,4-lactone)-5-yloxy, and the other of $R_3'$ and $R_4'$ represents hydroxy, lower alkoxy of 1 to 4 carbon atoms, pyridylmethoxy, benzyloxy or pivaloyloxymethoxy, and pharmaceutically acceptable salts thereof.

Further preferred as mono-or di-(ester or amide) prodrug derivatives of the compounds of formula IV wherein $R_3'$ and $R_4'$ represent hydroxy are the compounds of formula IV wherein either one or both of $R_3'$ and $R_4'$ represent (lower alkyl, lower alkoxy, halogen)-substituted 3-phthalidoxy, or especially 3-phthalidoxy, and pharmaceutically acceptable salts thereof.

Of greatest interest as mono- or di-(ester or amide) prodrug derivatives of the compounds of formula IV wherein $R_3'$ and $R_4'$ represent hydroxy are the compounds of formula IV wherein either one or both, especially one, of $R_3'$ and $R_4'$ represent 3-phthalidoxy, 1,2 : 3,4-di-O-isopropylidene-D-galactopyranos-6-yloxy, 1,2 : 5,6-di-O-isopropylidene-D-glucofuranos-3-yloxy, 2,3-isopropylidene-D-ribono-(1,4-lactone)-5-yloxy or 2,3-O-benzylidene-D-ribono-(1,4-lactone)-5-yloxy, and pharmaceutically acceptable salts thereof.

The compounds of the invention, of formulae I-IV and derivatives, may contain several asymmetric carbon atoms, depending on the nature of the substituents. Thus they exist in the form of stereoisomers, e. g., racemates, pure enantiomers, or mixtures thereof, all of which are within the scope of the invention. For example, the compounds of formula I (wherein R and/or $R_0$ represent substituents other than hydrogen) exist in isomeric forms, e. g. wherein the asymmetric carbon atoms bearing the R and $R_0$ groups may exist either in the S or R configuration.

The compounds of the invention, e. g. those of formula I having said two asymmetric centers exist as two distinct racemic diastereoisomeric forms which may be called erythro and threo depending on the relative orientation of the R and $R_0$ substituents on the chain. Each of the two racemates consists of the optically active enantiomers (or antipodes) having the (S, S), (R, R) and (R, S), (S, R) configurations, respectively.

Preferred are the threo racemic compounds and particularly the enantiomeric form thereof depicted in formula Ia

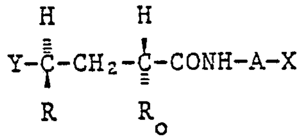
(Ia)

and wherein A, X, Y, R, $R_0$ have meaning as defined herein above for compounds of formula I.

For the glutaric acid derivatives of formula II (and esters and amides thereof) and of formula III and IV wherein both $R_1$ and $R_2$ (or $R_1'$ and $R_2'$) represent substituents other than hydrogen, the glutaryl chain likewise exists in two distinct diastereoisomeric forms which may be called erythro and threo respectively. Preferred are e. g. the compounds of formula IV as the threo diastereoisomer (racemate), more particularly as the enantiomeric form wherein the glutaryl portion is as depicted in formula IVa

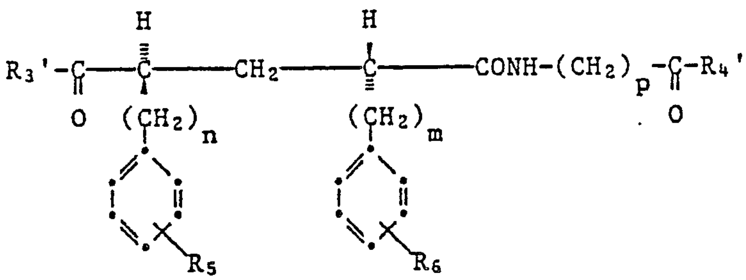
(IVa)

and wherein $R_3'$, $R_4'$, $R_5$, $R_6$, n, m and p have meaning as defined hereinabove for compounds of formula IV.

Illustrative thereof, in the above compounds of formula IVa wherein m and n represent the integer 1, each of the two carbon atoms on the glutaryl chain carrying said substituent is assigned the (S)-configuration ; in said compounds of formula IVa wherein m and n represent an integer from 2 to 4, each of the two carbon atoms is assigned the (R)-configuration.

Above all are preferred the compounds of formula I described in the examples and pharmaceutically acceptable salts thereof.

The compounds of the invention of formula I are prepared using conventional chemical methodology as applied to e. g. the following process which comprises : condensing a compound of formula V

$$NH_2—A—X \quad (V)$$

wherein A and X have meaning as defined above, in temporarily protected form if required, with a compound of formula VI

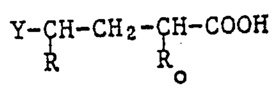
(VI)

or a reactive functional derivative thereof, wherein R, $R_0$ and Y have meaning as defined above, in temporarily protected form if required ; and, if temporarily protecting any interfering reactive group(s), removing said protecting group(s) ; and, if desired, converting a resulting compound of the invention into another compound of the invention, and/or, if desired, converting a resulting free compound into a salt or a resulting salt into the free compound or into another salt, and/or, if desired, separating a mixture of isomers or racemates obtained into the single isomers or racemates, and/or, if desired, resolving a racemate obtained into the optical antipodes.

In starting compounds and intermediates therefore which are converted to the compounds of the invention in a manner described herein, functional groups present, such as carboxy, amino and hydroxy groups, are optionally protected by conventional protecting groups that are common in preparative organic chemistry. The need and choice of protecting groups for a particular reaction is known to those skilled in the art and depends on the nature of the functional group to be protected (carboxy group, amino group, etc.), the structure and stability of the molecule of which the substituent is a part, and the reaction conditions. Well-known protecting groups that meet these conditions and their introduction and removal are described, for example, in J.F.W. McOmie, « Protective Groups in Organic Chemistry », Plenum Press, London, New York 1973 ; T.W. Greene, « Protective Groups in Organic Synthesis », Wiley, New York 1981, and also in « The Peptides », Vol. I, Schroeder and Luebke, Academic Press, London, New York 1965.

The condensation of an amine of formula V with the acid of formula VI, or a functional reactive

derivative thereof, is carried out by methodology well-known in the art.

Reactive functional derivatives of compounds of formula VI are preferably halides, mixed anhydrides such as the pivaloyl, alkoxycarbonyl or cyanoacetyl anhydride, cyclic glutaric anhydrides for compounds of formula VI where Y represents carboxy or the corresponding lactones for compounds of formula VI in which Y represents hydroxymethyl.

The condensation of a compound of formula V in suitably protected form depending on nature of substituents, with a compound of formula VI in the form of a free carboxylic acid is carried out advantageously in the presence of a condensing agent such as dicyclohexylcarbodiimide or 1,1'-diimidazolylcarbonyl in an inert solvent such as methylene chloride, preferably at room temperature or at a temperature near the boiling point of the solvent.

The condensation of a compound of formula V with the anhydride, as a reactive functional derivative of a compound of formula VI wherein Y represents carboxy, is carried out in an inert solvent such as toluene or methylene chloride, advantageously in the presence of a base, e. g. an inorganic base such as potassium carbonate or an organic base such as triethylamine or pyridine, at a temperature ranging from about 0° to 100°, preferably at room temperature.

The condensation of a compound of formula V with a reactive functional derivative of an acid of formula VI in the form of an acid halide, advantageously an acid chloride, or mixed anhydride, is carried out in an inert solvent under conditions analogous to those described above for condensation with a glutaric acid anhydride, advantageously in the presence of a basic solvent, e. g. pyridine.

The starting materials of formulae V and VI are known, or, if new, may be prepared according to conventional methods, e. g., those illustrated by the examples herein.

For example, starting materials of formula VI, e. g. wherein Y represents carboxy or functionally modified carboxy, are prepared from the correspondingly substituted glutaric anhydride by hydrolysis, alcoholysis or aminolysis by methods well known in the art for opening of a cyclic anhydride. Monofunctional derivatives of a dicarboxylic acid of formula VI (wherein Y does not represent free carboxy) are converted to the corresponding reactive functional derivative, e. g. an acyl halide, by treatment with e. g. oxalyl chloride in methylene chloride.

The starting substituted glutaric anhydride is prepared by cyclization of the correspondingly substituted glutaric acid by treatment with e. g. acetyl chloride.

The substituted glutaric acids are prepared by methods well-known in the art, e. g. by condensation of the appropriately substituted di-lower alkyl malonate with an optionally alpha-substituted acrylic acid derivative or precursor thereof, e. g. as illustrated in Acta Chem. Scand. 1958, 314 for the preparation of dibenzylglutaric acid.

In the case of 2,4-disubstituted glutaric acids, both threo and erythro diastereoisomers are obtained and may be isolated. When both the 2- and 4-substituents are identical, the isomers consist of the racemic (d, l) threo and meso erythro isomers. The racemic (d, l) diastereoisomer can be further resolved into the individual enantiomers by methods well-known in the art.

The compounds of the invention so obtained, can be converted into each other according to conventional methods. Thus, for example, resulting amides or esters may be hydrolyzed with aqueous alkalies, such as alkali metal carbonates or hydroxides. Resulting free acids may be esterified with e. g. said unsubstituted or substituted alkanols or reactive esterified derivatives thereof such as alkylhalides, or diazoalkanes, or free acids are also converted into said metal, ammonium or acid addition salts in conventional manner.

Thus, any resulting free acid or base can be converted into a corresponding metal, ammonium or acid addition salt respectively, by reacting it with an equivalent amount of the corresponding base, basic salt, acid or ion exchange preparation, e. g. said acids with alkali or ammonium hydroxides or carbonates, or e. g. aminoalkyl esters with said inorganic or organic acids respectively. Any resulting salt may also be converted into the free compound, by liberating the latter with stronger acids or bases, respectively. In view of the close relationship between the free compounds and the salts thereof, whenever a compound of the invention, or intermediate, is referred to in this context, a corresponding salt is also intended, provided such is possible or appropriate under the circumstances.

The compounds, including their salts, may also be obtained in the form of their hydrates, or include other solvents used for the crystallization.

Furthermore, the mono- or bis-functional derivatives of, e. g., the dicarboxylic acids of formula II, wherein either one or both carboxy groups are esterified by identical or different radicals, may be prepared by condensing a said diacid, e. g. of formula II or a mono ester derivative thereof, with an esterifying agent of the formula VII

$$R_7—Z \quad \text{(VII)}$$

wherein Z represents hydroxy or a reactive esterified hydroxyl group ; and $R_7$ represents any of the ester radicals defined hereinabove.

An ester radical $R_7$ is e. g. alkyl, e. g. methyl, ethyl, n- or isopropyl or butyl ; substituted lower alkyl e. g. the ω-amino-, ω-(N-methyl or N,N-dimethyl) amino-, alpha-carboxy- or alpha-ethoxy-carbonyl-(ethyl, propyl or butyl) ; aryl-lower alkyl, e. g. benzyl, (methyl, methoxy, chloro)-substituted benzyl, or pyridylmethyl ;

lower alkanoyloxy-lower alkyl, e. g. pivaloyloxymethyl ; 3-phthalidyl or (methyl, methoxy, chloro)-substituted 3-phthalidyl, (hydroxy, lower alkanoyloxy, lower alkoxy)-substituted lower alkoxymethyl, e. g. β-(hydroxy, acetyloxy, methoxy)-ethoxymethyl ; bicycloalkyloxy-carbonyl-lower alkyl, e. g. unsubstituted or lower alkyl substituted bicyclo [2,2,1] heptyloxycarbonyl-lower alkyl, advantageously bornyloxycarbonylmethyl ; or 1-(lower alkoxycarbonyloxy)-lower alkyl, e. g. 1-(methoxy-, ethoxy- or propoxycarbonyloxy)-methyl, -ethyl or -propyl ; protected monosaccharidyl as defined above ; or protected polyhydroxyalkyl as defined above.

A reactive esterified hydroxyl group Z in a compound of the formula VII is a hydroxyl group esterified by a strong inorganic or organic acid. Corresponding Z groups are in particular halogen, for example chlorine, bromide or preferably iodine, also sulfonyloxy groups, such as lower alkyl- or arylsulfonyloxy groups, for example methane-, ethane-, benzene- or toluene-sulfonyloxy groups.

The esterification of the carboxyl groups, optionally in salt form, with a compound of formula VII wherein Z represents a reactive esterified hydroxyl group, is performed in a manner known per se, in the presence of for example an organic base, such as an organic amine, for example a tertiary amine, such as tri-lower alkylamine, for example trimethylamine, triethylamine or ethyl-di-isopropylamine, an N,N-di-lower-alkyl-aniline, for example N,N-di-methyl-aniline, a cyclic tertiary amine, such as an N-lower-alkylated morpholine, for example N-methyl-morpholine, a base of the pyridine type, for example pyridine, an inorganic base, for example hydroxides, carbonates, or hydrogen carbonates of alkali metals or alkaline-earth metals, for example sodium, potassium or calcium hydroxide, carbonate or hydrogen carbonate, or a quaternary ammonium base, such as a tetraalkylammonium hydroxide, carbonate or hydrogen carbonate, for example in which alkyl is e. g. methyl, ethyl, propyl, isopropyl, butyl, or the like, or an oxirane, for example a lower 1,2-alkylene oxide, such as ethylene oxide or propylene oxide.

The di-carboxylic acid, e. g. of the formula II, or a monoester thereof is preferably first converted into a salt of one of the stated organic or inorganic bases, especially into the sodium or potassium salt, and is then reacted with a compound of the formula VII. The compounds of formula VII are known or can be prepared by methods well-known to the art.

A compound of the formula VII wherein Z is a reactive esterified hydroxyl group can be prepared in situ. For example, a compound of the formula VII wherein Z is chloro can be converted by treatment with sodium iodide in a solvent, for example in acetone or acetonitrile, into a compound of the formula VII wherein Z is iodo ; or esterification can be carried out with a chloro compound of the formula VII in the presence of sodium iodide.

The esterification reaction of carboxylic acids of the invention is performed in a suitable inert solvent or solvent mixture, for example in dimethylformamide, a halogenated hydrocarbon e. g. methylene chloride, carbon tetrachloride or chlorobenzene, a ketone, e. g. acetone, an ester, e. g. ethyl acetate, or a nitrile, e. g. acetonitrile, or mixtures thereof, preferably at room temperature, or if necessary at a reduced or elevated temperature, advantageously at — 10° to + 40 °C, and/or in an inert-gas atmosphere, for example in a nitrogen atmosphere.

Esterification of a carboxylic acid with an alcohol of formula VII wherein Z represents hydroxy is carried out in a manner known per se, preferably in the presence of an acid catalyst e. g. sulfuric acid or boron trifluoride etherate preferably at an elevated temperature, advantageously ranging from about 40 °C to 100 °C.

The compounds of the invention wherein R and/or $R_0$, or $R_1$ and/or $R_2$ or $R_1'$ and/or $R_2'$ contain a phenyl ring, or the compounds of the invention wherein A represents phenylene, may be converted to the corresponding compounds of the invention containing a cyclohexyl or cyclohexylene ring, respectively. Such conversion is carried out e. g. by catalytic hydrogenation in the presence of a catalyst such as rhodium, nickel or platinum in a polar medium using procedures well-known in the art and as illustrated in the examples.

In case mixtures of geometrical or optical isomers of the above compounds of formulae I to VI are obtained, these can be separated into the single isomers by methods in themselves known, e. g., by fractional distillation, crystallization and/or chromatography. Racemic products can likewise be resolved into the optical antipodes, for example, by separation of diastereoisomeric salts thereof, e. g., for basic compounds by the fractional crystallization of d- or l-(tartrate, mandelate or camphorsulfonate) salts, or for acidic compounds by fractional crystallization of d- or l-(alphamethylbenzylamine, cinchonidine, cinchonine, quinine, quinidine, ephedrine, dehydroabietylamine, brucine or strychnine)-salts. Advantageously, the more active of the two antipodes is isolated.

The above-mentioned reactions are carried out according to standard methods, in the presence or absence of diluents, preferably such as are inert to the reagents and are solvents thereof, of catalysts, alkaline or acidic condensing or said other agents respectively and/or inert atmospheres, at low temperatures, room temperature or elevated temperatures, preferably near the boiling point of the solvents used, at atmospheric or superatmospheric pressure.

The invention further includes any variant of said processes, in which an intermediate product obtainable at any stage of the process is used as starting material and any remaining steps are carried out, or the process is discontinued at any stage thereof, or in which the starting materials are formed under the reaction conditions, or in which the reaction components are used in the form of their salts or optically pure antipodes. Mainly those starting materials should be used in said reactions, that lead to the

formation of those compounds indicated above as being preferred. For example, the compounds of formula IVa or the corresponding threo racemate, are those derived from the corresponding trans-2,4-disubstituted glutaric anhydride.

The pharmaceutical compositions according to the invention are those suitable for enteral, such as oral or rectal, transdermal and parenteral administration to mammals, including man, comprising an effective amount of a pharmacologically active compound of the invention or pharmaceutically acceptable salts thereof, alone or in combination with one or more pharmaceutically acceptable carriers.

The pharmacologically active compounds of the invention are useful in the manufacture of pharmaceutical compositions comprising an effective amount thereof in conjunction or admixture with excipients or carriers suitable for either enteral or parenteral application.

Preferred are tablets and gelatin capsules comprising the active ingredient together with a) diluents, e. g. lactose, dextrose, sucrose, mannitol, sorbitol, cellulose and/or glycine ; b) lubricants, e. g. silica, talcum, stearic acid, its magnesium or calcium salts and/or polyethyleneglycol ; for tablets also c) binders, e. g. magnesium aluminum silicate, starch paste, gelatin, tragacanth, methylcellulose, sodium carboxymethylcellulose and/or polyvinylpyrrolidone ; if desired, d) disintegrants, e. g. starches, agar, alginic acid or its sodium salt, or effervescent mixtures ; and/or e) absorbents, colorants, flavors and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, the compositions may also contain other therapeutically valuable substances. Said compositions are prepared according to conventional mixing, granulating or coating methods, respectively, and contain about 0.1 to 75 %, preferably about 1 to 50 %, of the active ingredient.

Suitable formulations for transdermal application include an effective amount of a compound of the invention with carrier. Advantageous carriers include absorbable pharmacologically acceptable solvents to assist passage through the skin of the host. Characteristically, transdermal devices are in the form of a bandage comprising a backing member, a reservoir containing the compound, optionally with carriers, optionally a rate controlling barrier to deliver the compound to the skin of the host at a controlled and predetermined rate over a prolonged period of time, and means to secure the device to the skin.

A unit dosage for a mammal of about 50 to 70 kg may contain between about 10 and 100 mg of the active ingredient. The dosage of active compound is dependent on the species of warm-blooded animal (mammal), the body weight, age and individual condition, and on the form of administration.

The following examples are intended to illustrate the invention and are not to be construed as being limitations thereon. Temperatures are given in degrees Centigrade. If not mentioned otherwise, all evaporations are performed under reduced pressure, preferably between about 20 and 130 mbar.

The prefixes R* and S* as used herein, e. g. when referring to a 2,4-disubstituted-4-carboxybutyryl grouping (a 2,4-disubstituted glutaryl grouping) or derivatized form thereof, are used to indicate the relative configuration of the two asymmetric centres in the racemic form. The prefixes R and S are used to indicate the absolute configuration at each asymmetric center in the enantiomeric form.

## Example 1

a) The solution of 0.21 g of 4-aminobutyric acid and 0.50 g of trans-2,4-dibenzylglutaric anhydride in 5 ml of pyridine and 5 ml of methylene chloride is stirred at room temperature overnight. The mixture is concentrated, the residue dissolved in ethyl acetate and the solution is washed with 1 N hydrochloric acid, saturated sodium chloride, dried over magnesium sulfate, concentrated, and the residue is recrystallized from ether to yield the N-[(R*, R*)-2,4-dibenzyl-4-carboxybutyryl]-4-aminobutyric acid, melting at 127-129° ; NMR ($Me_2SO$-$d_6$) δ 12.2 (2H, $CO_2\underline{H}$), 7.82 (t, 1H, J = 6 Hz, N$\underline{H}$), 7.26 and 7.28 (s, 10H, Ar-$\underline{H}$), 3.0 (t, 2H, J = 7 Hz, N-C$\underline{H}_2$), 2.6 (m, 6H), 2.1 (t, 2H, J = 8 Hz, —C$\underline{H}_2$$CO_2H$), 1.6 (m, 4H).

b) Similarly prepared from meso-2,4-dibenzylglutaric anhydride is N-[(R*, S*)-2,4-dibenzyl-4-carboxybutyryl]-4-aminobutyric acid melting at 124-126° ; NMR ($CDCl_3$) δ 11.7 (2H, $CO_2\underline{H}$), 7.3 (s, 11H, Ar-$\underline{H}$, N$\underline{H}$), 3.1 (m, 2H, NC$\underline{H}_2$), 2.7 (m, 6H), 2.1 (m, 2H).

c) Similarly prepared from 2S,4S-dibenzylglutaric anhydride is N-(2S,4S-dibenzyl-4-carboxybutyryl)-4-aminobutyric acid. Its disodium monohydrate melts at 108-113° (dec.) ; $[\alpha]_D^{25}$ = + 11.7° (c = 1.0 in methanol).

The starting materials are prepared as follows : 6.9 g of 2,4-dibenzylglutaric acid [Acta Chem. Scand. 12, 314 (1958)] is refluxed in 50 ml of acetyl chloride for 3 hours, concentrated, diluted with 25 ml of toluene and evaporated to give a mixture of meso (cis)- and racemic (trans)-2,4-dibenzylglutaric anhydride. The residue is dissolved in 8.5 ml of toluene and 1.5 ml of triethylamine and heated to 50° until all the solid dissolves. The solution is left to stand overnight. The solid is collected to yield the trans-2,4-dibenzylglutaric anhydride melting at 153-155°. Heating under reflux with dioxan/water (1 : 1) overnight yields racemic (R*, R*)-dibenzylglutaric acid melting at 150-152°.

The racemic trans-2,4-dibenzylglutaric anhydride can also be isolated directly from the mixture of meso cis- and racemic trans-2,4-dibenzylglutaric anhydride by fractional crystallization with toluene. The mother liquors are then concentrated and upon standing crystallization occurs. After stirring with cyclohexane the solid is collected to yield the (cis)-meso-2,4-dibenzylglutaric anhydride melting at 55-57°.

The 2S,4S-dibenzylglutaric anhydride [m.p. 172-174° ; $[\alpha]_D^{25}$ = — 19.1° (c = 1 in $CHCl_3$)] is prepared similarly by refluxing 2S,4S-dibenzylglutaric acid in acetyl chloride for 3 hours, concentrating the mixture and recrystallizing from toluene.

The chiral 2S,4S-dibenzylglutaric acid is prepared as follows : to 5.0 g of R*, R*)-dibenzylglutaric acid in 20 ml of isopropanol is added 0.81 g of triethylamine and the mixture is stirred for 20 minutes. To this mixture is added 0.97 g of d(+)-α-methylbenzylamine in 20 ml of isopropanol and the solution is stirred overnight. The solid is collected and recrystallized twice from isopropanol to yield 2S,4S-dibenzylglutaric acid as the d(+)-α-methylbenzylamine salt melting at 201-203° ; $[\alpha]_D^{25}$ = — 20.4° (c = 1 in methanol). To a warm solution of the above salt in 70 ml of water and 30 ml of ethanol is added 1 ml of concentrated hydrochloric acid. After standing 24 hours the solid is collected, washed with water and dried to yield 2S,4S-dibenzylglutaric acid melting at 150-152° ; $[\alpha]_D^{25}$ = + 8.9° (c = 2.0 in methanol).

## Example 2

According to the methods illustrated by the previous example the following compounds are prepared :

a) N-[(R*, R*)-2,4-dibenzyl-4-carboxybutyryl]-3-aminopropionic acid, melting at 122-124°. N-[(R*, R*)-2,4-dibenzyl-4-carboxybutyryl]-3-aminopropionic acid is converted to the disodium salt by the following procedure : to the solution of 0.38 g of the diacid in 25 ml of methanol : water (2 : 1) is added 2.0 ml of 1.00 N sodium hydroxide. The solution is concentrated to give a solid. Ethanol is added and the suspension is concentrated and the solid is dried at 50° under high vacuum to yield the N-[(R*, R*)-2,4-dibenzyl-4-carboxybutyryl]-3-aminopropionic acid disodium salt.

b) N-(2S,4S-dibenzyl-4-carboxybutyryl)-3-aminopropionic acid, melting at 142-144°, $[\alpha]_D^{25}$ = + 25.6° (c = 2.0 in methanol) ;

c) N-[(R*,S*)-2,4-dibenzyl-4-carboxybutyryl]-3-aminopropionic acid isolated as the disodium salt melting at 250° (dec.) ; NMR (DMSO-$d_6$)-δ 8.1 (m, 1H, NH), 7.25 and 7.18 (s, 10H, Ar-H).

d) N-[(R*,R*)-2,4-dibenzyl-4-carboxybutyryl]-2-aminobenzoic acid melting at 175-177° ; NMR ($CDCl_3$) δ 11.9 (s, 2H, $CO_2\underline{H}$), 10.74 (s, 1H, N$\underline{H}$), 8.84 (d, 1H, J = 9 Hz), 8.18 (d, 1H, J = 9 Hz), 7.64 (t, 1H, J = 9 Hz), 7.27 and 7.20 (s, 11H), 2.9 (m, 6H), 2.0 (m, 2H).

e) N-[(R*,R*)-2,4-dibenzyl-4-carboxybutyryl]-3-aminobenzoic acid ; NMR ($CDCl_3$) δ 11.4 (s, 2H), 7.75 (m, 5H), 7.22 (s, 10H), 2.8 (m, 6H), 2.0 (m, 2H).

f) N-[(R*,R*)-2,4-dibenzyl-4-carboxybutyryl]-3-aminopropionitrile melting at 123-125°, from trans-2,4-dibenzylglutaric anhydride and 3-aminopropionitrile.

g) N-[(R*,R*)-2,4-dibenzyl-4-carboxybutyryl]-4-aminobenzoic acid melting at 234-236° ; NMR ($CDCl_3$) δ 12.3 (2H, $CO_2\underline{H}$), 10.3 (s, 1H, N$\underline{H}$), 7.95 (d, 2H, J = 10 Hz), 7.74 (d, 2H, J = 10 Hz), 7.3 (s, 10H), 2.72 (m, 6H), 1.8 (m, 2H) ; disodium salt has a melting point > 300°.

## Example 3

According to procedures illustrated by the previous examples are prepared :

a) N-[(R*,S*)-2,4-di(phenethyl)-4-carboxybutyryl]-3-aminopropionic acid isolated as the disodium salt melting at 260-265° (dec.) ; NMR ($D_2O$) δ 7.40 and 7.43 (s, 10H, Ar-H), 3.27 (m, 2H, $NCH_2$).

b) N-[(R*,R*)-2,4-di(phenethyl)-4-carboxybutyryl]-3-aminopropionic acid isolated as the disodium salt melting at 205-215° (dec.) ; NMR ($D_2O$) δ 7.40 and 7.43 (s, 10H, Ar-$\underline{H}$), 3.35 (m, 2H, $NC\underline{H}_2$).

The starting materials are prepared as follows :

5.0 g of (R*,S*)-2,4-di(phenethyl) glutaric acid is refluxed in 50 ml of acetyl chloride for 3 hours, concentrated, diluted with toluene and evaporated to give a solid. The residue is recrystallized from cyclohexane : toluene (3 : 1) to yield cis-2,4-di(phenethyl) glutaric anhydride melting at 79-81°. The trans-2,4-di(phenethyl) glutaric anhydride is prepared similarly from the (R*,R*) diacid.

The mixture of isomers of 2,4-di-(phenethyl) glutaric acid are prepared following the general

procedure described in Acta Chem. Scand. 12, 314 (1958). The reaction mixture is recrystallized from cyclohexane to yield (R*,S*)-2,4-di(phenethyl) glutaric acid melting at 129-132°. The mother liquor is concentrated and the residue is recrystallized twice from cyclohexane : toluene (3 : 2) to yield (R*,R*)-2,4-di(phenethyl) glutaric acid melting at 95-105°.

## Example 4

The suspension of 0.5 g of N-[(R*,R*)-2,4-dibenzyl-4-carboxybutyryl]-3-aminopropionic acid and 0.5 g of 5 % Rh/C in 10 ml of ethanol and 10 ml of water is hydrogenated for 36 hours at 3.4 bar pressure. The mixture is filtered through celite and concentrated. The colorless oil is treated with 1.0 N sodium hydroxyde in methanol and concentrated to yield N-[(R*,R*)-2,4-di-(cyclohexylmethyl)-4-carboxybutyryl]-3-aminopropionic acid as the disodium salt melting at 200-210° (dec.) ; NMR ($Me_2SO$-$d_6$) δ 8.14 (m, 1H, N<u>H</u>), 3.17 (m, 2H, $NC\underline{H}_2$).

## Example 5

a) The solution of 1.5 g of 3-aminopropionic acid and 1.8 g of sodium carbonate in 30 ml of water at 0° is added to 2.2 g of 4-ethoxycarbonyl-(R*,R*)-2,4-dibenzylbutyryl chloride and the mixture is stirred at room temperature for 4 hours. The mixture is extracted with ether, and the aqueous layer is acidified with 2N hydrochloric acid. The acidic solution is extracted with ethyl acetate, washed with saturated brine, dried (magnesium sulfate), filtered and concentrated. The residue is recrystallized from ether to yield N-[4-ethoxycarbonyl-(R*,R*)-2,4-dibenzylbutyryl]-3-amino-propionic acid, melting at 84-86° ; NMR ($CDCl_3$) δ 10.5 (1H, $CO_2\underline{H}$), 6.1 (t, 1H, J = 6 Hz, N<u>H</u>), 4.1 (q, 2H, J = 8 Hz, $OC\underline{H}_2$), 1.08 (t, 3H, J = 8 Hz, $C\underline{H}_3$).

b) Prepared similarly is N-[4-ethoxycarbonyl-(R*,R*)-2,4-dibenzylbutyryl]-4-aminobutyric acid obtained as a foam ; NMR ($CDCl_3$) δ 9.8 (1H, $CO_2\underline{H}$), 7.3 (10H, Ar-<u>H</u>), 5.68 (m, 1H, N<u>H</u>), 4.07 (q, 2H, J = 8 Hz, $OC\underline{H}_2$), 1.10 (t, 3H, J = 8 Hz, $C\underline{H}_3$).

The starting material is prepared as follows : 4.0 g of trans-2,4-dibenzyl glutaric anhydride is refluxed in 40 ml of ethanol : toluene (3 : 2) overnight. The reaction mixture is concentrated to yield 4-ethoxycarbonyl-(R*,R*)-2,4-dibenzylbutyric acid as an oil. Oxalyl chloride (3.5 ml) is added to the solution of 4.5 g of 4-ethoxycarbonyl-(R*,R*)-2,4-dibenzylbutyric acid in 10 ml of methylene chloride. The mixture is stirred at room temperature overnight and evaporated to yield 4-ethoxycarbonyl-(R*,R*)-2,4-dibenzylbutyryl chloride, which is used as such without further purification.

## Example 6

The solution of 2.37 g of N-[4-ethoxycarbonyl-(R*,R*)-2,4-dibenzylbutyryl]-3-aminopropionic acid, 0.61 g of 3-hydroxymethylpyridine and 1.15 g of dicyclohexylcarbodiimide in 25 ml of methylene chloride is stirred at room temperature for two days. The solid is removed, and the filtrate is diluted with 150 ml of ether and extracted with 0.5 N hydrochloric acid. The acidic aqueous layer is separated, basified and extracted with ethyl acetate. The organic layer is washed with saturated sodium chloride, dried over magnesium sulfate, filtered, concentrated and flash chromatographed on silica gel eluting with ethyl acetate : ethanol (9 : 1) to yield 3-pyridylmethyl N-[4-ethoxycarbonyl-(R*,R*)-2,4-dibenzylbutyryl]-3-amino-propionate as an oil ; NMR ($CDCl_3$) δ 8.70 (m, 2H), 7.77 (m, 1H), 7.28 (m, 11H), 5.56 (t, 1H, J = 7 Hz), 5.12 (s, 2H), 4.03 (q, 2H, J = 8 Hz), 1.08 (t, 3H, J = 8 Hz).

Treatment with ethanolic hydrogen chloride yields the hydrochloride salt.

## Example 7

Similarly to the procedure described in example 6, condensation of N-[4-ethoxycarbonyl-(R*,R*)-2,4-dibenzylbutyryl]-3-aminopropionic acid with morpholine in the presence of dicyclohexylcarbodiimide yields N-[N'-(4-ethoxycarbonyl-(R*,R*)-2,4-dibenzylbutyryl)-3-aminopropionyl]-morpholine as an oil ; NMR ($CDCl_3$) δ 7.28 and 7.22 (s, 10H) ; 5.74 (t, 1H, J = 7 Hz), 4.03 (q, 2H, J = 8 Hz), 1.08 (t, 3H, J = 8 Hz).

## Example 8

a) To a solution of 150 mg of N-(4-methoxycarbonyl-2-phenethylbutyryl)-4-aminobutyric acid methyl ester in 3 ml of methanol is added 1.0 ml of 1 N sodium hydroxide and stirred for 4 hours at room temperature. The mixture is acidified with 1.5 ml of 1 N hydrochloric acid, and concentrated. The solid is collected and washed with water to yield N-(4-carboxy-2-phenethylbutyryl)-4-aminobutyric acid.

b) Similarly prepared is N-(4-carboxy-4-phenethylbutyryl)-4-aminobutyric acid, from N-(4-methoxycarbonyl-4-phenethylbutyryl)-4-aminobutyric acid methyl ester.

The starting materials are prepared as follows : 1.90 g of 4-aminobutyric acid and 4.0 g of 2-phenethylglutaric anhydride (U.S. Pat. 4 374 847) in 20 ml of pyridine is heated at 80° overnight. The mixture is concentrated, the residue is dissolved in ethyl acetate and the solution is washed with 1 N hydrochloric acid, saturated sodium chloride, dried over magnesium sulfate and concentrated to give an oil. The mixture of isomeric diacids is dissolved in 100 ml of methanol containing 5 drops of concentrated sulfuric acid and refluxed for 20 hours. The mixture is concentrated, diluted with methylene chloride, washed with saturated sodium bicarbonate, dried over magnesium sulfate, concentrated, chromatographed eluting with 40 % ethyl acetate : hexane to yield N-(4-methoxycarbonyl-2-phenethylbutyryl)-4-aminobutyric acid methyl ester and N-(4-methoxycarbonyl-4-phenethylbutyryl)-4-aminobutyric acid methyl ester as individual isomers.

## Example 9

4-(Pivaloyloxymethoxycarbonyl)-(R*,R*)-2,4-dibenzylbutyryl chloride (1.2 g) in 10 ml of methylene chloride is added slowly to a solution of 1.15 g of benzyl 3-aminopropionate p-toluene-sulfonic acid salt and 0.46 ml of triethylamine in 20 ml of methylene chloride. The reaction is stirred overnight at room temperature. The solution is washed with 2 N hydrochloric acid, then with saturated sodium bicarbonate, dried over magnesium sulfate, filtered and concentrated to give N-[4-pivaloyloxymethoxycarbonyl-(R*,R*)-2,4-dibenzylbutyryl]-3-aminopropionic acid benzyl ester.

The starting material is prepared as follows :

A mixture of 4.0 g of 2,4-trans-dibenzylglutaric anhydride and 1.5 ml of benzyl alcohol in 15 ml of toluene is stirred at 80° for 16 hours to give 4-(benzyloxycarbonyl)-(R*,R*)-2,4-dibenzylbutyric acid.

The above acid (3.2 g) is treated with 3.8 ml of 2.1 N potassium hydroxide. The solution is evaporated. Toluene (100 ml) is added and the mixture is evaporated to give the potassium 4-(benzyloxycarbonyl)-(R*,R*)-2,4-dibenzylbutyrate.

To chloromethyl pivalate (1.13 g) in 25 ml of acetone is added sodium iodide (1.11 g). The reaction mixture is stirred at room temperature for 3 hours. Then it is filtered and the filtrate is evaporated. To the residue in 25 ml of dimethylformamide is added potassium 4-(benzyloxycarbonyl)-(R*,R*)-2,4-dibenzylbutyrate (3.3 g) in 25 ml of dimethylformamide. The reaction mixture is stirred at room temperature for 18 hours and then evaporated. The residue is dissolved in 150 ml of ether and washed with 3 × 50 ml of 10 % aqueous sodium bicarbonate and 3 × 50 ml of saturated aqueous sodium chloride. The organic layer is dried over magnesium sulfate and evaporated to give pivaloyloxymethyl 4-(benzyloxycarbonyl)-(R*,R*)-2,4-dibenzylbutyrate.

A solution of 2.5 g of pivaloyloxymethyl 4-(benzyloxycarbonyl)-(R*,R*)-2,4-dibenzylbutyrate in 75 ml of ethanol is hydrogenated at atmospheric pressure in the presence of 0.2 g of 5 % palladium on carbon. The reaction is filtered and evaporated to give 4-(pivaloyloxymethoxycarbonyl)-2,4-(R*,R*)-dibenzylbutyric acid.

To 1.8 g of the above acid in 30 ml of methylene chloride at room temperature is added 1.2 ml of oxalyl chloride. The reaction mixture is stirred at room temperature for 2.5 hours and then evaporated to give 4-(pivaloyloxymethoxycarbonyl)-(R*,R*)-2,4-dibenzylbutyryl chloride.

## Example 10

0.92 g of N-[4-pivaloyloxymethoxycarbonyl-(R*,R*)-2,4-dibenzylbutyryl]-3-aminopropionic acid benzyl ester in 20 ml of ethanol is hydrogenated at atmospheric pressure in the presence of 0.1 g of 5 % palladium on carbon. The reaction is filtered and evaporated to give N-(4-pivaloyloxymethoxycarbonyl-(R*,R*)-2,4-dibenzylbutyryl)-3-aminopropionic acid.

## Example 11

a) To the solution of 0.52 g of 3-amino-1-propanol in 10 ml of methylene chloride is added 0.5 g of 4-ethoxycarbonyl-(R*,R*)-2,4-dibenzylbutyryl chloride in 10 ml of methylene chloride. The mixture is stirred 10 minutes at room temperature. The reaction mixture is concentrated and diluted with ethyl acetate. The organic portions are washed with 1 N hydrochloric acid, saturated sodium bicarbonate and saturated sodium chloride. The organic layer is dried over magnesium sulfate, filtered and concentrated to give N-[4-ethoxycarbonyl-(R*,R*)-2,4-dibenzylbutyryl]-3-amino-1-propanol as a colorless oil ; NMR ($CDCl_3$) δ 7.31 and 7.28 (s, 10H, Ar-H), 5.82 (t, 1H, J = 7 Hz, NH), 4.02 (q, 2H, J = 8 Hz, $OCH_2$), 3.3-1.25 (m, 15H), 1.10 (t, 3H, J = 8 Hz, $CH_3$).

b) Similarly prepared from 3-aminoproprionitrile is the N-[4-ethoxycarbonyl-(R*,R*)-2,4-dibenzylbutyryl]-3-aminopropionitrile ; NMR ($CDCl_3$) δ 7.3 (m, 10H), 4.0 (q, 2H, J = 8 Hz), 3.7-1.7 (multiplets).

## Example 12

a) To a solution of 0.01 mole of 3-aminopropionic acid and 0.01 mole of 4-ethoxycarbonyl-(R*,R*)-2,4

dibenzylbutyric acid in 40 ml of methylene chloride are added 0.01 mole of 1-(3-dimethylaminopropyl)-3-ethylcarbodiimide hydrochloride and 0.01 mole of triethylamine. The reaction mixture is stirred at room temperature for 3 days. Diethyl ether (250 ml) is added and the mixture is washed with 25 ml of water, 25 ml of 2N aqueous hydrochloric acid and 25 ml of saturated aqueous sodium bicarbonate solution. The organic layer is dried over magnesium sulfate and evaporated to give N-[4-ethoxycarbonyl-(R*,R*)-2,4-dibenzylbutyryl]-3-aminopropionic acid, the compound of example 5a).

b) To a solution of 0.012 mole of 1,1'-carbonyldiimidazole in 20 ml of methylene chloride at 0° is added a solution of 0.01 mole of 4-ethoxycarbonyl-(R*,R*)-2,4-dibenzylbutyric acid in 20 ml of methylene chloride. After 1 hour 0.01 mole of 4-aminobutyric acid in 10 ml of pyridine is added dropwise over a period of 15 minutes. The reaction mixture is stirred at room temperature overnight. The reaction mixture is evaporated to dryness. The residue is dissolved in 75 ml of methylene chloride and washed with 2 × 25 ml of 2 N aqueous hydrochloric acid. The organic layer is dried over magnesium sulfate and evaporated to give after recrystallization from ether N-[4-ethoxycarbonyl-(R*,R*)-2,4-dibenzylbutyryl]-4-aminobutyric acid, the compound of example 5b).

## Example 13

According to methods similar to those described in the previous examples are prepared the following compounds of formula III wherein A represents ethylene or propylene, $R_1'$ and $R_2'$ are identical, and $R_4$ represents hydroxy, as the threo isomers.

| Compound | $R_1'$ and $R_2'$ | $R_3$ |
|---|---|---|
| 13/1 | benzyl | 1-bornyloxycarbonylmethoxy |
| 13/2 | benzyl | 3-phthalidoxy |
| 13/3 | benzyl | 1-(ethoxycarbonyloxy)-ethoxy |
| 13/4 | benzyl | 1-(ethoxycarbonyl)-ethoxy |
| 13/5 | p-chlorobenzyl | hydroxy |
| 13/6 | p-cyanobenzyl | hydroxy |
| 13/7 | p-trifluoromethylbenzyl | hydroxy |
| 13/8 | p-methylbenzyl | hydroxy |
| 13/9 | benzyl | 3-pyridylmethoxy |
| 13/10 | benzyl | amino |
| 13/11 | benzyl | dodecylamino |
| 13/12 | benzyl | hexadecyloxy |
| 13/13 | benzyl | cholest-5-en-3β-oxy |
| 13/14 | methyl | ethoxy |

Starting materials for compounds 13/1 through 13/4 are 1-bornyl iodoacetate, 3-bromophthalide, 1-chloroethyl ethyl carbonate, and ethyl lactate respectively. Starting material for compound 13/13 is cholesterol.

## Example 14

According to methods similar to those described in the previous examples are prepared the following compounds of formula II.

| Compound | $R_1$ and $R_2$ | A |
|---|---|---|
| 14/1 | benzyl | $-CH(-CH_3)-CH_2-$ |
| 14/2 | 2-(3-thienyl)-ethyl | $-CH_2CH_2-$ |
| 14/3 | 2-(2-thienyl)-ethyl | $-CH_2CH_2CH_2-$ |

(Continued)

| Compound | $R_1$ and $R_2$ | A |
|---|---|---|
| 14/4 | 3-pyridylmethyl | $-CH_2CH_2-$ |
| 14/5 | 2-(2-naphthyl)-ethyl | $-CH_2CH_2-$ |
| 14/6 | 4-biphenylmethyl | $-CH_2CH_2CH_2-$ |
| 14/7 | 2-biphenylmethyl | $-CH_2CH_2CH_2-$ |
| 14/8 | benzyl | 1,4-cyclohexylene |
| 14/9 | phenethyl ($C_6H_5CH_2CH_2-$) | 1,3-cyclohexylene |
| 14/10 | cyclopentylmethyl | $-CH_2CH_2CH_2-$ |
| 14/11 | benzyl | $-CH_2-CH(p\text{-}ClC_6H_4)-CH_2-$ |

Compound 14/11 is isolated as the disodium salt, melting at 138-149°.

Example 15

Preparation of an injectable formulation containing 25 mg of the active ingredient per 5 ml of solution :

Formula

| | |
|---|---|
| N-(R*,R*)-2,4-Dibenzyl-4-carboxybutyryl-3-aminopropionic acid | 25.0 g |
| Sodium bicarbonate | 5.5 g |
| Propylparaben | 1.0 g |
| Water for injection q.s. | 5 000.0 ml |

The active ingredient, sodium bicarbonate and preservative are dissolved in 3 500 ml of water for injection and the solution is diluted to 5 000 ml. The solution is filtered through a sterile filter and filled into injection vials under sterile conditions each vial containing 5 ml of the solution.

Example 16

Preparation of 10,000 capsules each containing 20 mg of the active ingredient :

Formula

| | |
|---|---|
| N-[4-Ethoxycarbonyl-(R*,R*)-2,4-dibenzylbutyryl]-3-aminopropionic acid | 200.00 g |
| Lactose | 1,790.0 g |
| Magnesium stearate | 10.0 g |

The powders are passed through a screen with openings of 0.6 mm. Then the drug substance is placed in a suitable mixer and mixed with the lactose and magnesium stearate until homogeneous. No. 3 capsules are filled with 200 mg using a capsule filling machine.

Analogously, injectable formulations or capsules are prepared from the remaining compounds of the invention, e. g., those illustrated by the examples herein.

Example 17

According to methods similar to those described in the previous examples are prepared the following compounds of formula IVa (of S,S-configuration) wherein m and n represent the integer 1, p is the integer 2, and $R_5$ and $R_6$ represent hydrogen. Unless otherwise indicated $[\alpha]_D^{25}$ is measured in methanol (c = 1.0).

(See Tables pages 17-19)

| Compound | $R_3'$ and $R_4'$ | m.p. | $[\alpha]_D^{25}$ |
|---|---|---|---|
| 17/1 | $R_3'$=1,2:5,6-di-O-isopropylidene-D-glucofuranos-3-yloxy; $R_4'$=OH. | 68-71° | +1.0 |
| 17/2 | $R_3'$=1,2:3,4-di-O-isopropylidene-D-galactopyranos-6-yloxy; $R_4'$=OH. | 63-65° | -4.7° |
| 17/3 | $R_3'$=2,3-O-isopropylidene-D-ribono-(1,4-lactone)-5-yloxy; $R_4'$=OH. | 150-152° | +2.4° |
| 17/4 | $R_3'$ and $R_4'$ = cholest-5-en-3β-oxy | 72-75° | -20.8° ($CHCl_3$) |
| 17/5 | $R_3'$=(2,2-dimethyl-1,3-dioxolan-4-yl)-methoxy; $R_4'$=OH; sodium salt. | 63-66° | +17.3° |
| 17/6 | $R_3'$=OH; $R_4'$=2,3-O-isopropylidene-D-ribono-(1,4-lactone)-5-yloxy. | 106-109° | -1.0° |
| 17/7 | $R_3'$=OH; $R_4'$=2,3:5,6-di-O-cyclohexylidene-D-mannofuranos-1-yloxy; sodium salt. | 90-97° | +27.6° |
| 17/8 | $R_3'$=OH; $R_4'$=2,3-O-cyclohexylidene-D-ribono-(1,4-lactone)-5-yloxy. | 109-112° | + 9.8° |
| 17/9 | $R_3'$=OH; $R_4'$=1,2:3,4-di-O-isopropylidene-D-galactopyranos-6-yloxy. | 70-75° | -12.4° |
| 17/10 | $R_3'$=OH; $R_4'$=1,2:5,6-di-O-isopropylidene-D-glucofuranos-3-yloxy. | 67-70° | +11° |
| 17/11 | $R_3'$=OH; $R_4'$=1-methyl-2,3-O-isopropylidene-β-D-ribofuranos-5-yloxy; sodium salt. | 40-50° | -11.8° |

| Compound | $R_3$' and $R_4$' | m.p. | $[\alpha]_D^{25}$ |
|---|---|---|---|
| 17/12 | $R_3$'=n-butoxy; $R_4$'=4-pyridyl-methoxy. | IR 1729 $cm^{-1}$ | +19.3° |
| 17/13 | $R_3$'=2,3-O-isopropylidene-D-ribono-(1,4-lactone)-5-yloxy; $R_4$'=4-pyridylmethoxy | 153-155° | +9.3° |
| 17/14 | $R_3$'=2-(dimethylamino)ethylamino; $R_4$'=1,2:5,6-di-O-isopropylidene-D-glucofuranos-3-yloxy. | 85-89° | +35° |
| 17/15 | $R_3$'=OH; $R_4$'=cholest-5-en-3β-oxy. | 152-154° | +9.6° |
| 17/16 | $R_4$'=OH; $R_3$'=cholest-5-en-3β-oxy. | 134-139° | +10° |
| 17/17 | $R_4$'=OH; $R_3$'=ethoxy. | 79-81° | +18° |
| 17/18 | $R_4$'=OH; $R_3$=pivaloyloxymethoxy; sodium salt. | 97-104° | |
| 17/19 | $R_3$'=2-(dimethylamino)ethylamino; $R_4$'=cholest-5-en-3β-oxy. | 142-146° | +33.4° |
| 17/20 | $R_3$'=n-butoxy; $R_4$'=2,3-O-iso-propylidene-D-ribono-(1,4-lactone)-5-yloxy. | (oil) | -6.21° |
| 17/21 | $R_3$'=1,2:3,4-di-O-isopropylidene-D-galactopyranos-6-yloxy; $R_4$'=4-pyridylmethoxy. | 41-44° | +9.2° (c=0.5) |
| 17/22 | $R_3$'=OH; $R_4$'=1,2-O-isopropylidene-D-glucofuranos-3-yloxy; sodium salt. | 70-75° | +5.1° |
| 17/23 | $R_3$'=OH; $R_4$=2,3:4,6-di-O-iso-propylidene-α-L-sorbofuranos-1-yloxy; sodium salt. | 71-76° | +1.3° |

| Compound | $R_3$' and $R_4$' | m.p. | $[\alpha]_D^{25}$ |
|---|---|---|---|
| 17/24 | $R_3$'=ethoxy; $R_4$'=1,2:5,6-di--O-isopropylidene--D-glucofuranos-3-yloxy. | oil | +5.4° |
| 17/25 | $R_3$'=ethoxy; $R_4$'=2,3-O-benzylidene-D-ribono-(1,4-lactone)-5-yloxy. | 37-40° | -13.2° |
| 17/26 | $R_3$'=2,3-O-benzylidene-D-ribono-(1,4-lactone)-5-yloxy; $R_4$'=OH | 144-147° | + 0,4° |
| 17/27 | $R_3$'=OH; $R_4$'=D-ribono-(1,4-lactone)-5-yloxy. | 47-50° | + 27.0° |
| 17/28 | $R_3$'=OH; $R_4$'=1,2:5,6-di-O-isopropylidene-D-allofuranos-3-yloxy. | 84-88° | +71.7° |
| 17/29 | $R_3$'=OH; $R_4$'=1,2-O-isopropylidene-5,6-bis-O-acetyl-D-glucofuranos-3-yloxy. | 95-100° | +19.0° |
| 17/30 | $R_3$'=OH; $R_4$'=1,2:5,6-di-O-cyclohexylidene-D-glucofuranos-3-yloxy. | 107-110° | + 8.4° |
| 17/31 | $R_3$'=1,2-O-isopropylidene-D-glucofuranos-3-yloxy; $R_4$'=OH. | 90-96° | + 1.5° |
| 17/32 | $R_3$'=3-phthalidoxy; $R_4$'=OH | 46-49° | +14.0° |
| 17/33 | $R_3$'=OH, $R_4$'=2,3-O-benzylidene-D-ribono-(1,4-lactone)-5-yloxy. | 64-67° | -9.0° |

Illustrative procedures

a) The compound of Example 17/21 is prepared as follows :

To the solution of 0.85 g of 4-[1,2 : 3,4-di-O-isopropylidene-D-galactopyranos-6-yloxy-carbonyl]-(S,S)-2,4-dibenzylbutyric acid and 0.4 g of 4-pyridylmethyl 3-aminopropionate dihydrochloride salt (prepared as described in Austr. J. Chem., 1978, 31, 1865) and 0.32 g of triethylamine in 5 ml of methylene chloride is added 0.31 g of 1-(3-dimethylaminopropyl)-3-ethylcarbodiimide hydrochloride. The mixture is stirred overnight, diluted with ethyl acetate, washed with saturated sodium bicarbonate, water, dried ($Na_2SO_4$), filtered and concentrated. The residue is flash chromatographed on silica gel eluting with ethyl acetate to

give 4-pyridylmethyl N-[4-(1,2:3,4-di-O-isopropylidene-D-galactopyranos-6-yloxy-carbonyl)-(S,S)-2,4-dibenzylbutyryl]-3-aminopropionate melting at 41-44° ;

$$[\alpha]_D^{25} = + 9.2° \text{ (c = 0.5 in methanol).}$$

b) The starting material for compound of Example 17/1, its benzyl ester, is prepared as follows :

To the solution of 1.6 g of N-[(S,S)-2,4-dibenzyl-4-carboxybutyryl]-3-aminopropionic acid benzyl ester and 0.88 g of diacetone-D-glucose (1,2 : 5,6-di-O-isopropylidene-D-glucofuranose) in 20 ml of methylene chloride is added 0.74 g of 1-(3-dimethylaminopropyl)-3-ethylcarbodiimide hydrochloride and 0.41 g of 4-dimethylaminopyridine. The mixture is stirred at room temperature overnight, diluted with ethyl acetate and washed with 1N hydrochloric acid, water, saturated sodium bicarbonate, water, dried ($Na_2SO_4$), filtered and concentrated.

The residue is flash chromatographed on silica gel eluting with ethyl acetate : methylene chloride (1 : 4) to give N-[4-(1,2 : 5,6-di-O-isopropylidene-D-glucofuranos-3-yloxy-carbonyl)-(S,S)-2,4-dibenzylbutyryl]-3-aminopropionic acid benzyl ester as a colorless foam.

c) The starting material for compound of Example 17/2, its benzyl ester, is prepared as follows : the solution of 1 g of (S,S)-2,4-dibenzylglutaric anhydride and 1.06 g of 1,2 : 3,4-di-O-isopropylidene-D-galactopyranose in 10 ml of toluene is refluxed overnight. The solution is diluted with toluene, washed with water, dried ($Na_2SO_4$), filtered, and concentrated to give 4-[1,2 : 3;4-di-O-isopropylidene-D-galactopyranos-6-yloxycarbonyl]-(S,S)-2,4-dibenzylbutyric acid as a viscous oil.

To the solution of 0.85 g of 4-(1,2 : 3,4-di-O-isopropylidene-D-galactopyranos-6-yloxy-carbonyl)-(S,S)-2,4-dibenzylbutyric acid,

0.56 g of 3-aminopropionic acid benzyl ester p-toluenesulfonate and

0.16 g of triethylamine in 15 ml of methylene chloride is added

0.31 g of 1-(3-dimethylaminopropyl)-3-ethylcarbodiimide hydrochloride. The solution is stirred at room temperature overnight. The solution is diluted with ethyl acetate, washed with 1N hydrochloric acid, water, saturated sodium bicarbonate, water, dried ($Na_2SO_4$), filtered and concentrated. The residue is flash chromatographed on silica gel eluting with ethyl acetate : methylene chloride (1 : 4) giving N-[4-(1,2 : 3,4-di-O-isopropylidene-D-galactopyranos-6-yloxycarbonyl)-(S,S)-2,4-dibenzylbutyryl]-3-aminopropionic acid benzyl ester as a glass.

The cited esters, either as starting materials or final products, are prepared using the alcohols corresponding to $R_3'$ and/or $R_4'$, which are either commercially available, known in the literature or prepared according to known methods, e. g.

a) 2,3 : 5,6-di-O-cyclohexylidene-α-D-mannofuranose, J. Chem. Soc. 853 (1959) ;
b) 2,3-O-isopropylidene-D-ribono-1,4-lactone, Can. J. Chem. 1720 (1958) ;
c) 2,3 : 4,6-di-O-isopropylidene-α-L-sorbofuranose, Biochemistry 26, 201 (1971) ;
d) 1-methyl-2,3-O-isopropylidene-D-ribofuranoside ;
e) 2,2-dimethyl-1,3-dioxolane-4-methanol ;
f) 1,2 : 5,6-di-O-isopropylidene-D-glucofuranose ;
g) 1,2 : 5,6-di-O-isopropylidene-α-D-allofuranose ;
h) 1,2 : 3,4-di-O-isopropylidene-D-galactopyranose ;
i) 1,2-O-isopropylidene-D-glucofuranose ;
j) cholest-5-en-3β-ol,
k) phthalide.

### Example 18

According to procedures essentially as described in previous examples are prepared :

a) N-[(R*,R*)-2,4-di(p-methylbenzyl)-4-carboxybutyryl]-3-aminopropionic acid, isolated as the disodium salt, melting at 290-295° (dec.) ; see Example 13/8.

The starting material, racemic (R*,R*)-2,4-di(p-methylbenzyl)-glutaric acid, is prepared according to the procedure described in Example 1 for 2,4-dibenzylglutaric acid using corresponding p-methyl substituted starting materials.

b) N-[(S,S)-2,4-dibenzyl-4-ethoxycarbonylbutyryl]-3-amino-(N'-dodecyl)-propionamide, melting at 72-77°.

c) N-[(R*,R*)-2,4-dibenzyl-4-carboxybutyryl]-cis-2-aminocyclohexanecarboxylic acid, m. p. 145-160°.
d) N-[(S,S)-2,4-dimethyl-4-carboxybutyryl]-3-aminopropionic acid disodium salt, m. p. 125-129°.

### Example 19

According to precedures essentially as described in the previous examples are prepared the

following compounds of formula IVa (of S,S-configuration) wherein m and n represent the integer 1, p is 3, $R_5$ and $R_6$ represent hydrogen and

a) $R_3'$ = OH ;
$R_4'$ = 2,3-O-isopropylidene-D-ribono-(1,4-lactone)-5-yloxy, isolated as the sodium salt, melting at 94-104° (dec.) ;
$[\alpha]_D^{25}$ = + 5.9° (c = 1.0 in methanol) ;

b) $R_3'$ = OH ; $R_4'$ = D-ribono-(1,4-lactone)-5-yloxy ; $[\alpha]_D^{25}$ = + 15.2° (c = 1.0 in methanol) ;

c) $R_3'$ = ethoxy ; $R_4'$ = 2,3-O-benzylidene-D-ribono-(1,4-lactone)-5-yloxy ;

d) $R_3'$ = OH ; $R_4'$= 2,3-O-benzylidene-D-ribono-(1,4-lactone)-5-yloxy, m. p. 61-65° ; $[\alpha]_D^{25}$ = — 14.8° (c = 0.5 in methanol) ;

e) $R_3'$ = OH ; $R_4'$ = 1,2 : 3,4-di-O-isopropylidene-D-galactopyranos-6-yloxy, m. p. 62-68° ; $[\alpha]_D^{25}$ = — 11.8° (c = 1.0 in methanol) ;

f) $R_3'$ = OH ; $R_4'$ = 2,3-O-cyclohexylidene-D-ribono-(1,4-lactone)-5-yloxy ; isolated as the sodium salt, melting at 99-104° ; $[\alpha]_D^{25}$ = + 11.8° (c = 1.0 in methanol) ;

g) $R_3'$ = OH ; $R_4'$ = 1,2 : 5,6-di-O-cyclohexylidene-D-glucofuranos-3-yloxy ; m. p. 58-64° ; $[\alpha]_D^{25}$ = + 5.5° (c = 1.0 in methanol) ;

h) $R_3'$ = OH ; $R_4'$ = 1,2 : 5,6-di-O-isopropylidene-D-glucofuranos-3-yloxy ; m. p. 60-64° ; $[\alpha]_D^{25}$ = + 1.0° (c = 1.0 in methanol) ;

i) $R_3'$ = ethoxy ; $R_4'$ = 2,3-O-isopropylidene-D-ribono-(1,4-lactone)-5-yloxy ; oil ; $[\alpha]_D^{25}$ = — 8.6° (c = 1.0 in methanol) ;

j) $R_3'$ = OH ; $R_4'$= 2,3-O-cyclohexylidene-D-ribono-(1,4-lactone)-5-yloxy ; m. p. 99-104° ; $[\alpha]_D^{25}$ = + 11.8° (c = 1.0 in methanol) ;

Example 20

The solution of 300 mg of N-[(S,S)-2,4-dibenzyl-4-carboxybutyryl]-3-aminopropionic acid 1,2 : 5,6-di-O-isopropylidene-D-glucofuranos-3-yl ester (compound of Example 17/10) in 9 ml of 80 % aqueous acetic acid is stirred at room temperature for 24 hours. The mixture is concentrated. Residual acetic acid is removed by addition of toluene and concentrating the solution ; a colorless oil is obtained. The resulting N-[(S,S)-2,4-dibenzyl-4-carboxybutyryl]-3-aminopropionic acid 1,2-O-isopropylidene-D-glucofuranos-3-yl ester is isolated as the sodium salt melting at 70-75° (compound of Example 17/22).

**Claims**

1. Compounds of formula I

$$Y-\underset{R}{CH}-CH_2-\underset{R_0}{CH}-CONH-A-X \qquad (I)$$

wherein X and Y independently represent hydroxymethyl ; cyano ; carboxy ; functionally modified carboxy selected from esterified carboxy, carbamoyl, and N-substituted carbamoyl ; 5-tetrazolyl ; 2-oxazolyl, 4,5-dihydro-2-oxazolyl, 2-imidazolyl or 4,5-dihydro-2-imidazolyl or any said grouping substituted by lower alkyl ; R and $R_0$ independently represent lower alkyl, ($C_3$-$C_7$)-cycloalkyl-lower alkyl, or aryl-lower alkyl in which aryl represents phenyl, pyridyl, thienyl, furyl, biphenylyl or naphthyl, each unsubstituted or mono- or di-substituted by halogen, lower alkyl, hydroxy, acyloxy, lower alkoxy, trifluoromethyl or cyano ; A represents straight chain ($C_2$-$C_5$)-alkylene ; or A represents straight chain ($C_2$-$C_5$)-alkylene substituted by lower alkyl, by lower alkylthio-lower alkyl, by hydroxy-lower alkyl, by acyloxy-lower alkyl, by lower alkoxy-lower alkyl, by amino or acylamino, by amino-lower alkyl, by acylamino-lower alkyl, by ($C_3$-$C_7$)-cycloalkyl, by ($C_3$-$C_7$)-cycloalkyl-lower alkyl, by aryl or aryl-lower alkyl in which aryl represents phenyl or phenyl mono- or disubstituted by halogen, lower alkyl, lower alkoxy, hydroxy, acyloxy, trifluoromethyl or cyano ; or A represents phenylene or cyclohexylene ; or pharmaceutically acceptable prodrug derivatives of any said compounds having a free carboxy group ; or pharmaceutically acceptable salts of any said compounds with a salt-forming group.

2. Compounds of formula II according to claim 1

$$HOOC-\underset{R_1}{CH}-CH_2-\underset{R_2}{CH}-CONH-A-COOH \qquad (II)$$

wherein $R_1$ and $R_2$ independently represent lower alkyl, ($C_3$-$C_7$)-cycloalkyl-lower alkyl, or aryl-lower alkyl in which aryl represents phenyl, pyridyl, thienyl, furyl, biphenylyl or naphthyl each unsubstituted or mono- or di-substituted by halogen, lower alkyl, hydroxy, lower alkoxy, trifluoromethyl or cyano ; A represents straight chain ($C_2$-$C_5$)-alkylene ; or A represents straight chain ($C_2$-$C_5$)-alkylene substituted by lower alkyl, by lower alkylthio-lower alkyl, by hydroxy-lower alkyl, by acyloxy-lower alkyl, by lower alkoxy-lower alkyl, by amino or acylamino, by amino-lower alkyl, by acylamino-lower alkyl, by ($C_3$-$C_7$)-cycloalkyl, by ($C_3$-$C_7$)-cycloalkyl-lower alkyl, by aryl or aryl-lower alkyl in which aryl represents phenyl or phenyl mono- or disubstituted by halogen, lower alkyl, hydroxy, lower alkoxy, trifluoromethyl or cyano ; or A represents phenylene or cyclohexylene ; a mono- or bis-carboxylic acid derivative thereof in which the derivative is selected from an unsubstituted amide or mono- or di-($C_1$-$C_{20}$)-alkylamide ; a tertiary lower alkylene, oxalkylene or azaalkylene amide wherein the lower alkylene, oxalkylene or azaalkylene group together with the amide nitrogen forms a 5-, 6- or 7-membered ring, or said lower alkylene, oxalkylene or azaalkylene amide is substituted on the ring by lower alkyl, hydroxy-lower alkyl or by lower alkanoyloxy-lower alkyl ; an alpha-lower alkoxycarbonyl- or alpha-carboxy-substituted lower alkylamide ; an alpha-lower alkoxycarbonyl- or alpha-carboxy-substituted aryl-lower alkylamide in which aryl represents optionally substituted phenyl as defined above or 3-indolyl ; an (amino or acylamino)-lower alkylamide ; a ($C_1$-$C_{20}$)-alkyl ester ; an (amino, acylamino, mono- or di-lower alkylamino, carboxy or lower alkoxycarbonyl)-substituted lower alkyl ester ; an aryl-lower alkyl ester in which aryl represents optionally substituted phenyl as defined above or pyridyl ; a lower alkanoyloxy-lower alkyl ester ; a phthalidyl ester ; a (hydroxy, lower alkanoyloxy, or lower alkoxy)-substituted lower alkoxymethyl ester ; a bicycloalkyloxycarbonyl-lower alkyl ester having up to 10 carbon atoms in the bicycloalkyl group ; a hydroxyamide ; a lower alkylsulfonylamide ; or a 1-(lower alkoxycarbonyloxy)-lower alkyl ester ; a 3-cholestanyl or 3-cholestenyl ester ; and also a monosaccharidyl or protected monosaccharidyl ester being an ester incorporating as the alcohol portion a monosaccharide or protected monosaccharide, e. g. a free or protected aldopentose, aldohexose, ketopentose or ketohexose, in straight chain or cyclic form, e. g. furanose or pyranose form, or a free or protected glyconic acid of 5 or 6 carbon atoms or a lactone thereof ; a polyhydroxy-lower alkyl or protected polyhydroxy-lower alkyl ester being an ester incorporating as the alcohol portion a polyhydroxy-lower alkane or protected polyhydroxy-lower alkane, e. g. a free or protected glycerol or erythritol ; and pharmaceutically acceptable salts of any said compound with a salt-forming group.

3. Compounds of formula III according to claim 1

$$R_3-\overset{O}{\overset{\|}{C}}-\underset{R_1'}{CH}-CH_2-\underset{R_2'}{CH}-CONH-A-\overset{O}{\overset{\|}{C}}-R_4 \qquad (III)$$

wherein $R_1'$ and $R_2'$ independently represent lower alkyl or aryl-($C_1$-$C_4$)-alkyl in which aryl represents phenyl or phenyl mono- or di-substituted by halogen, lower alkyl, hydroxy, lower alkoxy, trifluoromethyl or cyano ; A represents straight chain ($C_2$-$C_5$)-alkylene ; or A represents straight chain ($C_2$-$C_5$)-alkylene mono-substituted by lower alkyl, by phenyl or phenyl-lower alkyl, by (halogen, lower alkyl, hydroxy, trifluoromethyl or lower alkoxy)-mono- or di-substituted phenyl or phenyl-lower alkyl, by lower alkylthio-lower alkyl, by hydroxy-lower alkyl, by lower alkoxy-lower alkyl, by amino-lower alkyl or by acylamino-lower alkyl ; or A represents phenylene or cyclohexylene ; in which $R_3$ and $R_4$ independently represent hydroxy, ($C_1$-$C_{20}$)-alkoxy ; (amino, acylamino, mono- or di-lower alkylamino)-lower alkoxy ; carboxy-lower alkoxy ; lower alkoxycarbonyl-lower alkoxy ; aryl-lower alkoxy in which aryl represents optionally (halogen, lower alkyl, hydroxy or lower alkoxy)-mono- or di-substituted phenyl or pyridyl ; (hydroxy, lower alkanoyloxy or lower alkoxy)-lower alkoxy ; (hydroxy, lower alkanoyloxy or lower alkoxy)-lower alkoxymethoxy ; bicyclo [2,2,1]-heptyloxycarbonyl-lower alkoxy ; cholestan-3-oxy or cholest-5-en-oxy ; 3-phthalidoxy ; furthermore, (lower alkyl, lower alkoxy, halogen)-substituted 3-phthalidoxy, also monosaccharidyloxy or protected monosaccharidyloxy representing e. g. glucosyloxy, galactosyloxy, mannosyloxy, sorbosyloxy, allosyloxy, ribosyloxy, arabinosyloxy, ribonyloxy, gluconyloxy, or cyclic, e. g. appropriate pyranose, furanose or lactone forms thereof, wherein hydroxy groups are free or one or more, as appropriate, are protected in form of esters, e. g., a lower alkanoyl or a benzoyl ester, in form of ethers, e. g. a lower alkyl or benzyl ether, or, in case two vicinal hydroxy groups are involved, in the form of acetals or ketals, e. g. a lower alkylidene, a benzylidene or a 5- or 6-membered cycloalkylidene derivative ; and also polyhydroxy-lower alkoxy or protected polyhydroxy-lower alkoxy representing, e. g., dihydroxypropyloxy or trihydroxybutyloxy wherein hydroxy groups are free or one or more, as appropriate, are protected in form of esters, e. g., a lower alkanoyl or a benzoyl ester, in form of ethers, e. g. a lower alkyl or benzyl ether, or, in case two vicinal hydroxy groups are involved, in the form of acetals or ketals, e. g. a lower alkylidene, a benzylidene or a 5- or 6-membered cycloalkylidene derivative ; and also 1-(lower

alkoxycarbonyloxy)-lower alkoxy ; amino ; mono- or di-($C_1$-$C_{20}$)-alkylamino ; morpholino ; N-lower alkylpiperazino ; pyrrolidino ; piperidino ; perhydroazepino ; (amino or acylamino)-lower alkylamino ; alpha-(carboxy or lower alkoxycarbonyl)-lower alkylamino ; aryl-lower alkylamino in which aryl is phenyl or 3-indolyl and which can be substituted on the alpha-carbon by carboxy or lower alkoxycarbonyl ; hydroxyamino ; or lower alkylsulfonylamino ; and pharmaceutically acceptable salts of any said compounds with a salt forming group.

4. Compounds of formula III according to claim 3 wherein $R_1'$ and $R_2'$ independently represent aryl-($C_1$-$C_4$)-alkyl in which aryl represents phenyl or phenyl mono- or di-substituted by halogen, lower alkyl, hydroxy, lower alkoxy, trifluoromethyl or cyano ; A represents straight chain ($C_2$-$C_5$)-alkylene, straight chain ($C_2$-$C_5$)-alkylene substituted by lower alkyl, phenyl, halophenyl, or substituted by phenyl-lower alkyl ; or A represents phenylene or cyclohexylene ; $R_3$ and $R_4$ independently represent hydroxy ; ($C_1$-$C_{20}$)-alkoxy ; (amino, mono- or di-lower alkylamino)-lower alkoxy ; alpha-carboxy-lower alkoxy ; alpha-lower alkoxycarbonyl-lower alkoxy ; aryl-methoxy in which aryl represents phenyl, pyridyl, or (halogen, lower alkyl or lower alkoxy)-monosubstituted phenyl or pyridyl ; (lower alkanoyloxy or lower alkoxy)-methoxy ; (hydroxy, lower alkanoyloxy or lower alkoxy)-ethoxymethoxy ; bicyclo [2,2,1] heptyloxycarbonylmethoxy ; 1-(lower alkoxycarbonyloxy)-lower alkoxy ; furthermore, 3-phthalidoxy or (lower alkyl, lower alkoxy, halogen)-substituted 3-phthalidoxy, cholestan-3-oxy or cholest-5-en-3-oxy ; also monosaccharidyloxy or protected monosaccharidyloxy selected from glucosyloxy, galactosyloxy, mannosyloxy, sorbosyloxy, allosyloxy, ribosyloxy, arabinosyloxy, ribonyloxy, gluconyloxy, or cyclic, e. g. appropriate pyranose, furanose or lactone forms thereof, wherein hydroxy groups are free or one or more, as appropriate, are protected in form of a lower alkanoyl or a benzoyl ester, in form of a lower alkyl or benzyl ether, or, in case two vicinal hydroxy groups are involved, in the form of a lower alkylidene, a benzylidene or a 5- or 6-membered cycloalkylidene derivative ; polyhydroxy-lower alkoxy or protected polyhydroxy-lower alkoxy selected from dihydroxypropyloxy or trihydroxybutyloxy wherein hydroxy groups are free or one or more, as appropriate, are protected in form of a lower alkanoyl or a benzoyl ester, in form of a lower alkyl or benzyl ether, or, in case two vicinal hydroxy groups are involved, in the form of a lower alkylidene, a benzylidene or a 5- or 6-membered cycloalkylidene derivative ; and also amino ; mono- or di-($C_1$-$C_{20}$)-alkylamino ; morpholino ; N-methylpiperazino ; piperidino ; perhydroazepino ; amino-lower alkylamino ; alpha-(carboxy or lower alkoxycarbonyl)-lower alkylamino ; phenyl-alpha-(carboxy or lower alkoxycarbonyl)-lower alkylamino ; hydroxyamino ; or lower alkylsulfonylamino ; and pharmaceutically acceptable salts of any said compounds with a basic or acidic salt-forming group.

5. Compounds of formula III according to claim 3 wherein $R_3$ and $R_4$ independently represent hydroxy, ($C_1$-$C_{20}$)-alkoxy, pivaloyloxymethoxy, bornyloxycarbonylmethoxy, benzyloxy, pyridylmethoxy alpha-carboxyethoxy, alpha-lower alkoxycarbonylethoxy, 3-phthalidoxy, furthermore monosaccharidyloxy or protected monosaccharidyloxy selected from glucofuranosyloxy, glucopyranosyloxy, galactopyranosyloxy, allofuranosyloxy, mannofuranosyloxy, ribofuranosyloxy, sorbofuranosyloxy, arabinofuranosyloxy and ribono-(1,4-lactone)-yloxy, wherein hydroxy groups are free or hydroxy groups are protected in form of a lower alkanoyl ester, in form of a benzyl ether or in form of an isopropylidene, a benzylidene or cyclohexylidene derivative ; and also amino, mono- or di-lower alkylamino, morpholino, alpha-(carboxy or lower alkoxycarbonyl)-lower alkylamino, or lower alkylsulfonylamino ; and pharmaceutically acceptable salts olf any said compounds with a basic or acid salt-forming group.

6. Compounds of formula III according to claim 3 in which the groups $COR_3$ and $COR_4$ represent independently carboxy or carboxy esterified in form of a pharmaceutically acceptable ester wherein $R_3$ and $R_4$ independently represent hydroxy, lower alkoxy, benzyloxy, pyridylmethoxy, pivaloyloxymethoxy, 3-phthalidoxy, also monosaccharidyloxy or protected monosaccharidyloxy.

7. Compounds of formula III according to claim 3 wherein one of $R_3$ and $R_4$ represents hydroxy and the other of $R_3$ and $R_4$ represents protected monosaccharidyloxy.

8. Compounds of formula III according to claim 3 wherein one of $R_3$ and $R_4$ represents hydroxy and the other of $R_3$ and $R_4$ represents 3-phthalidoxy.

9. Compounds of formula IV according to claim 1

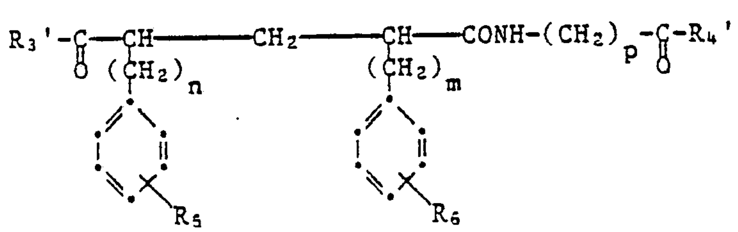

(IV)

wherein m and n independently represent an integer from 1 to 4 ; p represents an integer from 2 to 4 ; $R_3'$ and $R_4'$ represent hydroxy ; $R_5$ and $R_6$ independently represent hydrogen, halogen, hydroxy, lower alkoxy, lower alkyl or trifluoromethyl ; or a pharmaceutically acceptable mono- or di-(ester or amide) prodrug derivative thereof ; and pharmaceutically acceptable salts of any said compounds with a free carboxy group or basic salt-forming group.

10. Compounds of formula IV according to claim 9 wherein m and n independently represent an integer from 1 to 4 ; p represents an integer from 2 to 4 ; $R_3'$ and $R_4'$ independently represent hydroxy, ($C_1$-$C_{20}$)-alkoxy, amino, pivaloyloxymethoxy, bornyloxycarbonylmethoxy, benzyloxy, pyridylmethoxy, 1-(lower alkoxycarbonyloxy)-lower alkoxy or lower alkyl-sulfonylamino, furthermore 3-phthalidoxy ; and also monosaccharidyloxy or protected monosaccharidyloxy selected from glucofuranosyloxy, glucopyranosyloxy, galactopyranosyloxy, allofuranosyloxy, mannofuranosyloxy, ribofuranosyloxy, sorbofuranosyloxy, arabinofuranosyloxy and ribono (1,4-lactone)-yloxy, wherein hydroxy groups are free or hydroxy groups are protected in form of a lower alkanoyl ester, in form of a benzyl ether or in form of an isopropylidene, a benzylidene or cyclohexylidene lower derivative ; $R_5$ and $R_6$ independently represent hydrogen, halogen, hydroxy, lower alkoxy, lower alkyl or trifluoromethyl ; and pharmaceutically acceptable salts of any said compounds with a free carboxy group or basic salt-forming group.

11. The threo racemic compounds of formula I according to claim 1.

12. Compounds of formula I according to claim 1 being present as the enantiomeric form depicted in formula Ia

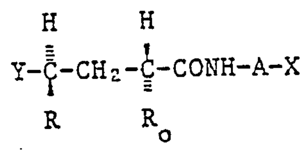

(Ia)

13. Compounds of formula IV according to claim 9 being present as the enantiomeric form depicted in formula IVa

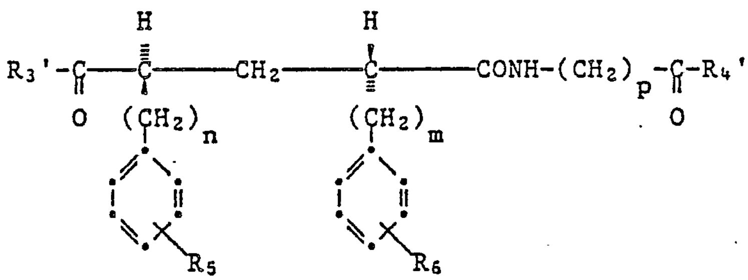

(IVa)

14. A compound of claim 11 being N-[(R*,R*)-2,4-dibenzyl-4-carboxybutyryl]-3-aminopropionic acid or N-[(R*,R*)-2,4-dibenzyl-4-carboxybutyryl]-4-aminobutyric acid ; a pharmaceutically acceptable prodrug mono- or diester derivative thereof ; the S,S-antipode of a said compound ; or a pharmaceutically acceptable salt of a said compound having an acid or basic salt forming group.

15. A compound of claim 9 being N-[4-ethoxycarbonyl-2,4-dibenzylbutyryl]-3-aminopropionic acid, a stereoisomer thereof, a mixture of different stereoisomers thereof ; or a pharmaceutically acceptable salt thereof.

16. A compound of claim 9 being N-[4-ethoxycarbonyl-2,4-dibenzylbutyryl]-4-aminobutyric acid, a stereoisomer thereof, a mixture of different stereoisomers thereof ; or a pharmaceutically acceptable salt thereof.

17. A compound of claim 9 being N-(2,4-dibenzyl-4-carboxybutyryl)-4-aminobutyric acid or N-(2,4-dibenzyl-4-carboxybutyryl)-3-aminopropionic acid, a stereoisomer thereof, mixtures of these stereoisomers and pharmaceutically acceptable salts thereof.

18. Pharmaceutical preparations containing a compound of formula I according to claim 1 or a pharmaceutically acceptable salt thereof.

19. A compound of the formula I according to claim 1 and pharmaceutically acceptable salts thereof for use in a method for the prophylactic and/or therapeutic treatment of the animal or human body.

20. A compound of the formula I according to claim 1 and pharmaceutically acceptable salts thereof as compounds that have analgesic activity.

21. A compound of the formula I according to claim 1 and pharmaceutically acceptable salts thereof as compounds that have antihypertensive activity.

22. Use of compounds of the formula I according to claim 1 or of pharmaceutically acceptable salts thereof for the manufacture of pharmaceutical preparations.

23. Process for the manufacture of compounds of the formula I according to claim 1, or salts thereof, which comprises : condensing a compound of formula V

$$NH_2—A—X \qquad (V)$$

wherein A and X have meaning as defined under formula I, in temporarily protected form if required, with a compound of formula VI

$$Y-\underset{R}{CH}-CH_2-\underset{R_o}{CH}-COOH \quad (VI)$$

or a reactive functional derivative thereof, wherein R, $R_0$ and Y have meaning as defined under formula I, in temporarily protected form if required ; and, if temporarily protecting any interfering reactive group(s), removing said protecting group(s) ; and, if desired, condensing a diacid of formula I obtained, wherein X and Y are carboxy, or a mono ester derivative thereof, with an esterifying agent of the formula VII

$$R_7—Z \quad (VII)$$

wherein Z represents hydroxy or a reactive esterified hydroxyl group ; and $R_7$ represents any of the ester radicals defined for formula I in order to obtain a mono- or bis-functional derivative of said diacid of formula I wherein either one or both carboxy groups are esterified by identical or different radicals, and, if desired, converting a resulting compound of the invention into another compound of the invention, and/or, if desired, converting a resulting free compound into a salt or a resulting salt into the free compound or into another salt, and/or, if desired, separating a mixture of isomers or racemates obtained into the single isomers or racemates, and/or, if desired, resolving a racemate obtained into the optical antipodes.

**Patentansprüche**

1. Verbindungen der Formel I

$$Y-\underset{R}{CH}-CH_2-\underset{R_o}{CH}-CONH-A-X \quad (I)$$

worin X und Y unabhängig voneinander Hydroxymethyl ; Cyano ; Carboxy ; funktionell abgewandeltes Carboxy ausgewählt aus der Gruppe verestertes Carboxy, Carbamoyl und N-substituiertes Carbamoyl ; 5-Tetrazolyl ; 2-Oxàzolyl, 4,5-Dihydro-2-oxazolyl, 2-Imidazolyl oder 4,5-Dihydro-2-imidazolyl, oder irgendeine der letztgenannten Gruppen substituiert durch Niederalkyl bedeuten ; R und $R_0$ unabhängig voneinander Niederalkyl, ($C_3$-$C_7$)-Cycloalkyl-niederalkyl, oder Arylniederalkyl, worin Aryl für Phenyl, Pyridyl, Thienyl, Furyl, Biphenylyl oder Naphthyl steht und unsubstituiert oder durch Halogen, Niederalkyl, Hydroxy, Acyloxy, Niederalkoxy, Trifluormethyl oder Cyano mono- oder disubstituiert ist, bedeuten ; A geradkettiges ($C_2$-$C_5$)-Alkylen bedeutet ; oder A für geradkettiges ($C_2$-$C_5$)-Alkylen, das durch Niederalkyl, Niederalkylthio-niederalkyl, Hydroxy-niederalkyl, Acyloxy-niederalkyl, Niederalkoxy-niederalkyl, Amino, Acylamino, Amino-niederalkyl, Acylamino-niederalkyl, ($C_3$-$C_7$)-Cycloalkyl, ($C_3$-$C_7$)-Cycloalkyl-niederalkyl, Aryl oder Arylniederalkyl, worin Aryl Phenyl oder Phenyl, welches durch Halogen, Niederalkyl, Niederalkoxy, Hydroxy, Acyloxy, Trifluormethyl oder Cyano mono- oder disubstituiert ist, bedeutet, substituiert ist, steht ; oder A Phenylen oder Cyclohexylen bedeutet ; oder pharmazeutische annehmbare Prodrug-Derivate von einer der oben genannten Verbindungen, die eine freie Carboxygruppe hat ; oder pharmazeutisch annehmbare Salze von einer der oben genannten Verbindungen mit einer salzbildenden Gruppe.

2. Verbindungen der Formel II gemäss Anspruch 1

$$HOOC-\underset{R_1}{CH}-CH_2-\underset{R_2}{CH}-CONH-A-COOH \quad (II)$$

worin $R_1$ und $R_2$ unabhängig voneinander Niederalkyl, ($C_3$-$C_7$)-Cycloalkylniederalkyl oder Aryl-niederalkyl, worin Aryl für Phenyl, Pyridyl, Thienyl, Furyl, Biphenylyl oder Naphthyl steht und unsubstituiert oder durch Halogen, Niederalkyl, Hydroxy, Niederalkoxy, Trifluormethyl oder Cyano mono- oder di-substituiert ist, bedeuten ; A geradkettiges ($C_2$-$C_5$)-Alkylen bedeutet ; oder A für geradkettiges ($C_2$-$C_5$)-Alkylen, das durch Niederalkyl, Niederalkylthio-niederalkyl, Hydroxy-niederalkyl, Acyloxyniederalkyl, Niederalkoxy-niederalkyl, Amino, Acylamino, Amino-niederalkyl, Acylamino-niederalkyl, ($C_3$-$C_7$)-Cycloalkyl, ($C_3$-$C_7$)-Cycloalkylniederalkyl, Aryl oder Aryl-niederalkyl, worin Aryl Phenyl oder Phenyl, welches durch Halogen, Niederalkyl, Hydroxy, Niederalkoxy, Trifluormethyl oder Cyano mono- oder disubstituiert ist, bedeutet, substituiert ist, steht ; oder A Phenylen oder Cyclohexylen bedeutet ; ein Mono- oder Dicarbonsäurederivat davon ausgewählt aus der Gruppe unsubstituiertes Amid oder Mono- oder Di($C_1$-$C_{20}$)-alkylamid ; tertiäres Niederalkylen-, Oxalkylen- oder Azaalkylenamid, worin die Niederalkylen-, Oxalkylen- oder Azaalkylengruppe zusammen mit dem Amidstickstoff einen 5-, 6- oder 7-gliedrigen Ring bildet, oder ein genanntes Niederalkylen-, Oxalkylen- oder Azaalkylenamid, welches durch Niederalkyl, Hydroxy-niederalkyl oder Niederalkanoyloxy-niederalkyl ringsubstituiert ist ; alpha-Niederalkoxycarbonyl- oder alpha-Carboxy-substituiertes Niederalkylamid ; alpha-Niederalkoxycarbonyl- oder alpha-Carboxy-substituiertes

Aryl-niederalkylamid, worin Aryl für gegebenenfalls substituiertes Phenyl wie oben definiert oder 3-Indolyl steht; (Amino oder Acylamino)-niederalkylamid; ($C_1$-$C_{20}$)-Alkylester; (Amino, Acylamino, Mono- oder Di-niederalkylamino, Carboxy oder Niederalkoxycarbonyl)-substituierter Niederalkylester; Aryl-niederalkylester, worin Aryl gegebenenfalls substituiertes Phenyl wie oben definiert oder Pyridyl bedeutet; Niederalkanoyloxy-niederalkylester; Phthalidylester; (Hydroxy, Niederalkanoyloxy oder Niederalkoxy)-substituierter Niederalkoxymethylester; Bicycloalkyloxycarbonyl-niederalkylester, der bis zu 10 Kohlenstoffatome in der Bicycloalkylgruppe trägt; Hydroxyamid; Niederalkylsulfonylamid; 1-(Niederalkoxycarbonyloxy)-niederalkylester; 3-Cholestanyl- oder 3-Cholestenylester; Monosaccharidylester oder geschützter Monosaccharidylester, worunter Ester zu verstehen sind, die als Alkoholteil ein Monosaccharid oder ein geschütztes Monosaccharid, z. B. eine freie oder geschützte Aldopentose, Aldohexose, Ketopentose or Ketohexose, geradkettig oder in cyclischer Form, z. B. Furanose- oder Pyranoseform, oder eine freie oder geschützte ($C_5$-$C_6$)-Glyconsäure oder ein Lacton davon enthalten; und Polyhydroxy-niederalkylester oder geschützter Polyhydroxy-niederalkylester, worunter Ester zu verstehen sind, die als Alkoholteil ein Polyhydroxy-niederalkan oder ein geschütztes Polyhydroxy-niederalkan, z. B. freies oder geschütztes Glycerin oder Erythritol, enthalten; und pharmazeutisch annehmbare Salze von irgendeiner der genannten Verbindungen mit einer salzbildenden Gruppe.

3. Verbindungen der Formel III gemäss Anspruch 1

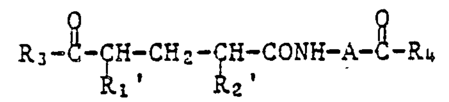 (III)

worin $R_1'$ und $R_2'$ unabhängig voneinander Niederalkyl oder Aryl-($C_1$-$C_4$)-alkyl bedeuten, worin Aryl für Phenyl oder Phenyl, das durch Halogen, Niederalkyl, Hydroxy, Niederalkoxy, Trifluormethyl oder Cyano mono- oder disubstituiert ist, steht; A geradkettiges ($C_2$-$C_5$)-Alkylen bedeutet; oder A geradkettiges ($C_2$-$C_5$)-Alkylen, das durch Niederalkyl, Phenyl, Phenyl-niederalkyl, (Halogen, Niederalkyl, Hydroxy, Trifluormethyl oder Niederalkoxy)-mono- oder disubstituiertes Phenyl oder Phenyl-niederalkyl, Niederalkylthio-niederalkyl, Hydroxy-niederalkyl, Niederalkoxy-niederalkyl, Amino-niederalkyl oder Acylamino-niederalkyl substituiert ist, bedeutet; oder A Phenylen oder Cyclohexylen bedeutet; worin $R_3$ und $R_4$ unabhängig voneinander Hydroxy, ($C_1$-$C_{20}$)-Alkoxy; (Amino, Acylamino, Mono- oder Di-niederalkylamino)-niederalkoxy; Carboxy-niederalkoxy; Niederalkoxycarbonyl-niederalkoxy; Aryl-niederalkoxy, worin Aryl für gegebenenfalls (Halogen, Niederalkyl, Hydroxy oder Niederalkoxy)-mono- oder disubstituiertes Phenyl oder Pyridyl steht; (Hydroxy, Niederalkanoyloxy oder Niederalkoxy)-niederalkoxy; (Hydroxy, Niederalkanoyloxy oder Niederalkoxy)-niederalkoxymethoxy; Bicyclo-[2,2,1]-heptyloxycarbonylniederalkoxy; Cholestan-3-oxy oder Cholest-5-en-oxy; 3-Phthalidoxy; (Niederalkyl, Niederalkoxy, Halogen)-substituiertes 3-Phthalidoxy, Monosaccharidyloxy oder geschütztes Monosaccharidyloxy, z. B. Glucosyloxy, Galactosyloxy, Mannosyloxy, Sorbosyloxy, Allosyloxy, Ribosyloxy, Arabinosyloxy, Ribonyloxy, Gluconyloxy, oder cyclische Formen davon, z. B. geeignete Pyranose-, Furanose- oder Lactonformen, worin die Hydroxygruppen frei sind oder eine oder mehrere in Form von Estern geschützt sind, z. B. als Niederalkanoyl-, oder Benzoylester, oder in Form von Ethern, z. B. als Niederalkyl- oder Benzylether, oder, im Falle von zwei vicinalen Hydroxygruppen, in Form von Acetalen oder Ketalen, z. B. als Niederalkyliden-, Benzyliden- oder 5- oder 6-gliedriges Cycloalkylidenderivat; Polyhydroxy-niederalkoxy oder geschütztes Polyhydroxy-niederalkoxy, z. B. Dihydroxypropyloxy oder Trihydroxybutyloxy, worin die Hydroxygruppen frei sind oder eine oder mehrere geschützt sind in Form von Estern, z. B. als Niederalkanoyl- oder Benzoylester, in Form von Ethern, z. B. als Niederalkyl- oder Benzylether, oder, im Falle von zwei vicinalen Hydroxygruppen, in Form von Acetalen oder Ketalen, z. B. als Niederalkyliden-, Benzyliden- oder 5- oder 6-gliedriges Cycloalkylidenderivat; 1-(Niederalkoxycarbonyloxy)-niederalkoxy; Amino; Mono- oder Di-($C_1$-$C_{20}$)-alkylamino; Morpholino; N-Niederalkylpiperazino; Pyrrolidino; Piperidino; Perhydroazepino; (Amino oder Acylamino)-niederalkylamino; alpha-(Carboxy oder Niederalkoxycarbonyl)-niederalkylamino; Aryl-niederalkylamino, worin Aryl Phenyl oder 3-Indolyl bedeutet, und welches am alpha-Kohlenstoff durch Carboxy oder Niederalkoxycarbonyl substituiert sein kann; Hydroxyamino; oder Niederalkylsulfonylamino; und pharmazeutisch annehmbare Salze von irgendeiner der genannten Verbindungen mit einer salzbildenden Gruppe.

4. Verbindungen der Formel III gemäss Anspruch 3, worin $R_1'$ und $R_2'$ unabhängig voneinander Aryl-($C_1$-$C_4$)-alkyl bedeuten, worin Aryl für Phenyl oder Phenyl, das durch Halogen, Niederalkyl, Hydroxy, Niederalkoxy, Trifluormethyl oder Cyano mono- oder disubstituiert ist, steht; A geradkettiges ($C_2$-$C_5$)-Alkylen oder geradkettiges ($C_2$-$C_5$)-Alkylen, das durch Niederalkyl, Phenyl, Halogenphenyl oder Phenyl-niederalkyl substituiert ist, bedeutet; oder A Phenylen oder Cyclohexylen bedeutet; $R_3$ und $R_4$ unabhängig voneinander Hydroxy; ($C_1$-$C_{20}$)-Alkoxy; (Amino, Mono- oder Di-niederalkylamino)-niederalkoxy; alpha-Carboxy-niederalkoxy; alpha-Niederalkoxycarbonyl-niederalkoxy; Aryl-methoxy, worin Aryl für Phenyl, Pyridyl oder (Halogen, Niederalkyl oder Niederalkoxy)-mono-substituiertes Phenyl oder Pyridyl steht; (Niederalkanoyloxy oder Niederalkoxy)-methoxy; (Hydroxy, Niederalkanoyloxy oder Niederalkoxy)-ethoxymethoxy; Bicyclo-[2,2,1] heptyloxycarbonylmethoxy; 1-(Niederalkoxycarbonyloxy)-niederalkoxy; 3-Phthalidoxy oder (Niederalkyl, Niederalkoxy, Halogen)-substituiertes 3-Phthalidoxy, Cholestan-3-oxy oder Cholest-5-en-3-oxy; Monosaccharidyloxy oder geschütztes Monosaccharidyloxy

ausgewählt aus der Gruppe Glucosyloxy, Galactosyloxy, Mannosyloxy, Sorbosyloxy, Allosyloxy, Ribosyloxy, Arabinosyloxy, Ribonyloxy, Gluconyloxy, oder cyclische, z. B. geeignete Pyranose-, Furanose- oder Lactonformen davon, worin die Hydroxygruppen frei sind oder eine oder mehrere geschützt sind in Form eines Niederalkanoyl- oder Benzoylesters, in Form eines Niederalkyl- oder Benzylethers, oder, im Falle von zwei vicinalen Hydroxygruppen, in Form eines Niederalkyliden-, Benzyliden- oder 5- oder 6-gliedrigen Cycloalkylidenderivats ; Polyhydroxy-niederalkoxy oder geschütztes Polyhydroxy-niederalkoxy ausgewählt aus der Gruppe Dihydroxypropyloxy oder Trihydroxybutyloxy, worin die Hydroxygruppen frei sind oder eine oder mehrere geschützt sind in Form eines Niederalkanoyl- oder Benzoylesters, in Form eines Niederalkyl- oder Benzylethers, oder, im Falle von zwei vicinalen Hydroxygruppen, in Form eines Niederalkyliden-, Benzyliden- oder 5- oder 6-gliedrigen Cycloalkylidenderivats ; Amino ; Mono- oder Di-($C_1$-$C_{20}$)-alkylamino ; Morpholino ; N-Methylpiperazino ; Piperidino ; Perhydroazepino ; Amino-niederalkylamino ; alpha-Carboxy oder Niederalkoxycarbonyl)-niederalkylamino ; Phenyl-alpha-(carboxy oder niederalkoxycarbonyl)-niederalkylamino ; Hydroxyamino ; oder Niederalkylsulfonylamino bedeuten ; und pharmazeutisch annehmbare Salze von irgendeiner der genannten Verbindungen mit einer basischen oder sauren salzbildenden Gruppe.

5. Verbindungen der Formel III gemäss Anspruch 3, worin $R_3$ und $R_4$ unabhängig voneinander Hydroxy, ($C_1$-$C_{20}$)-Alkoxy, Pivaloyloxymethoxy, Bornyloxycarbonylmethoxy, Benzyloxy, Pyridylmethoxy, alpha-Carboxyethoxy, alpha-Niederalkoxycarbonylethoxy, 3-Phthalidoxy, Monosaccharidyloxy oder geschütztes Monosaccharidyloxy ausgewählt aus der Gruppe Glucofuranosyloxy, Glucopyranosyloxy, Galactopyranosyloxy, Allofuranosyloxy, Mannofuranosyloxy, Ribofuranosyloxy, Sorbofuranosyloxy, Arabinofuranosyloxy und Ribono-(1,4-lacton)-yloxy, worin die Hydroxygruppen in freier Form vorliegen oder geschützt sind in Form eines Niederalkanoylesters, in Form eines Benzylethers oder in Form eines Isopropyliden-, Benzyliden- oder Cyclohexylidenderivats ; Amino, Mono- oder Di-niederalkylamino, Morpholino, alpha-(Carboxy oder Niederalkoxycarbonyl)-niederalkylamino, oder Niederalkylsulfonylamino bedeuten ; und pharmazeutisch annehmbare Salze von irgendeiner der genannten Verbindungen mit einer basischen oder sauren salzbildenden Gruppe.

6. Verbindungen der Formel III gemäss Anspruch 3, worin die Gruppen $COR_3$ and $COR_4$ unabhängig voneinander Carboxy oder verestertes Carboxy, welches als pharmazeutisch annehmbarer Ester vorliegt und worin $R_3$ und $R_4$ unabhängig voneinander für Hydroxy, Niederalkoxy, Benzyloxy, Pyridylmethoxy, Pivaloyloxymethoxy, 3-Phthalidoxy, Monosaccharidyloxy oder geschütztes Monosaccharidyloxy stehen, bedeuten.

7. Verbindungen der Formel III gemäss Anspruch 3, worin einer der Reste $R_3$ und $R_4$ Hydroxy bedeutet und der andere der Reste $R_3$ und $R_4$ geschütztes Monosaccharidyloxy bedeutet.

8. Verbindungen der Formel III gemäss Anspruch 3, worin einer der Reste $R_3$ und $R_4$ Hydroxy bedeutet und der andere der Reste $R_3$ und $R_4$ 3-Phthalidoxy bedeutet.

9. Verbindungen der Formel IV gemäss Anspruch 1

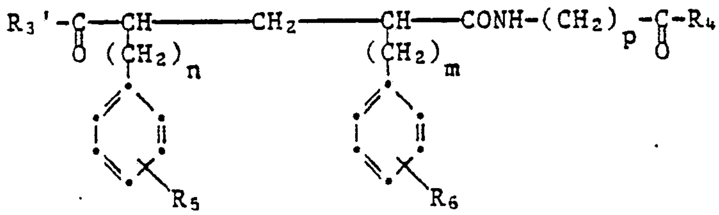

(IV)

worin m und n unabhängig voneinander eine ganze Zahl von 1 bis 4 bedeuten ; p eine ganze Zahl von 2 bis 4 bedeutet ; $R_3'$ und $R_4'$ Hydroxy bedeuten ; $R_5$ und $R_6$ unabhängig voneinander Wasserstoff, Halogen, Hydroxy, Niederalkoxy, Niederalkyl oder Trifluormethyl bedeuten ; oder ein pharmazeutisch annehmbares Mono- oder Di-(ester oder amid) Prodrug-Derivat davon ; und pharmazeutisch annehmbare Salze von irgendeiner der genannten Verbindungen mit einer freien Carboxygruppe oder einer basischen salzbildenden Gruppe.

10. Verbindungen der Formel IV gemäss Anspruch 9, worin m und n unabhängig voneinander eine ganze Zahl von 1 bis 4 bedeuten ; p eine ganze Zahl von 2 bis 4 bedeutet ; $R_3'$ und $R_4'$ unabhängig voneinander Hydroxy, ($C_1$-$C_{20}$)-Alkoxy, Amino, Pivaloyloxymethoxy, Bornyloxycarbonylmethoxy, Benzyloxy, Pyridylmethoxy, 1-(Niederalkoxycarbonyloxy)-niederalkoxy oder Niederalkylsulfonylamino, 3-Phthalidoxy ; Monosaccharidyloxy oder geschütztes Monosaccharidyloxy ausgewählt aus der Gruppe Glucofuranosyloxy, Glucopyranosyloxy, Galactopyranosyloxy, Allofuranosyloxy, Mannofuranosyloxy, Ribofuranosyloxy, Sorbofuranosyloxy, Arabinofuranosyloxy und Ribono(1,4-lacton)-yloxy, worin die Hydroxygruppen frei oder geschützt sind in Form eines Niederalkanoylesters, in Form eines Benzylethers oder in Form eines Isopropyliden-, Benzyliden- oder Cyclohexylidenderivats, bedeuten ; $R_5$ und $R_6$ unabhängig voneinander Wasserstoff, Halogen, Hydroxy, Niederalkoxy, Niederalkyl oder Trifluormethyl bedeuten ; und pharmazeutisch annehmbare Salze von irgendeiner der genannten Verbindungen mit einer freien Carboxygruppe oder einer basischen salzbildenden Gruppe.

11. Die threo-racemischen Verbindungen der Formel I gemäss Anspruch 1.

12. Verbindungen der Formel I gemäss Anspruch 1 in der in Formel Ia wiedergegebenen enantiomeren Form

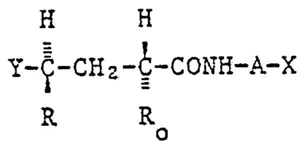

(Ia)

13. Verbindungen der Formel IV gemäss Anspruch 9 in der in Formel IVa wiedergegebenen enantiomeren Form

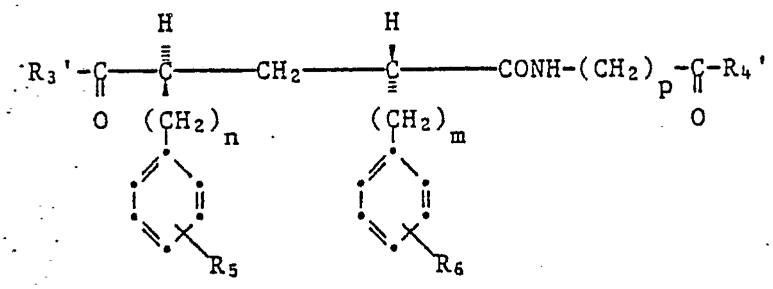

(IVa)

14. Eine Verbindung gemäss Anspruch 11 aus der Gruppe N-[(R*, R*)-2,4-Dibenzyl-4-carboxybutyryl]-3-aminopropionsäure und N-[(R*,R*)-2,4-Dibenzyl-4-carboxybutyryl]-4-aminobuttersäure ; ein pharmazeutisch annehmbares Prodrug Mono- oder Diesterderivat davon ; den S,S-Antipoden einer der genannten Verbindungen ; oder ein pharmazeutisch annehmbares Salz einer der genannten Verbindungen mit einer sauren oder basischen salzbildenden Gruppe.

15. N-[4-Ethoxycarbonyl-2,4-dibenzylbutyryl]-3-aminopropionsäure gemäss Anspruch 9, ein Stereoisomer davon, eine Mischung verschiedener Stereoisomere davon ; oder ein pharmazeutisch annehmbares Salz davon.

16. N-[4-Ethoxycarbonyl-2,4-dibenzylbutyryl]-4-aminobuttersäure gemäss Anspruch 9, ein Stereoisomer davon, eine Mischung verschiedener Stereoisomere davon ; oder ein pharmazeutisch annehmbares Salz davon.

17. Eine Verbindung gemäss Anspruch 9 aus der Gruppe N-(2,4-Dibenzyl-4-carboxybutyryl)-4-aminobuttersäure und N-(2,4-Dibenzyl-4-carboxybutyryl)-3-aminopropionsäure, ein Stereoisomer davon, eine Mischung aus diesen Stereoisomeren und pharmazeutisch annehmbare Salze davon.

18. Pharmazeutische Präparate enthaltend eine Verbindung der Formel I gemäss Anspruch 1 oder ein pharmazeutisch annehmbares Salz davon.

19. Eine Verbindung der Formel I gemäss Anspruch 1 und pharmazeutisch annehmbare Salze davon zur Verwendung in einem Verfahren zur prophylaktischen und/oder therapeutischen Behandlung des tierischen oder menschlichen Körpers.

20. Eine Verbindung der Formel I gemäss Anspruch 1 und pharmazeutisch annehmbare Salze davon als Verbindungen, die analgetische Wirksamkeit besitzen.

21. Eine Verbindung der Formel I gemäss Anspruch 1 und pharmazeutisch annehmbare Salze davon als Verbindungen, die antihypertensive Wirkung haben.

22. Verwendung von Verbindungen der Formel I gemäss Anspruch 1 oder von pharmazeutisch annehmbaren Salzen davon zur Herstellung von pharmazeutischen Präparaten.

23. Verfahren zur Herstellung von Verbindungen der Formel I gemäss Anspruch 1 oder Salzen davon, dadurch gekennzeichnet, dass man eine Verbindung der Formel V

$$NH_2—A—X \qquad (V)$$

worin A und X die unter Formel I angegebene Bedeutung haben, falls nötig in vorübergehend geschützter Form, mit einer Verbindung der Formel VI

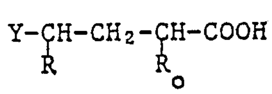

(VI)

oder einem reaktiven funktionellen Derivat davon, worin R, $R_0$ und Y die unter Formel I angegebene Bedeutung haben, falls nötig in vorübergehend geschützter Form, kondensiert ; und, dass man, falls reaktive Gruppen vorübergehend geschützt werden, die genannten Schutzgruppen entfernt ; und dass man, falls gewünscht, eine erhaltene Disäure der Formel I, worin X und Y Carboxy bedeuten, oder ein Monoesterderivat davon, mit einem veresternden Agens der Formel VII

$$R_7{-}Z \qquad (VII)$$

worin Z Hydroxy oder eine reaktive veresterte Hydroxygruppe bedeutet und $R_7$ irgendeinen der Esterreste, die für Formel I definiert sind, bedeutet, umsetzt, um ein mono- oder bifunktionales Derivat der genannten Disäure der Formel I zu erhalten, worin entweder eine oder beide Carboxygruppen durch gleiche oder verschiedene Reste verestert sind, und, falls gewünscht, dass man eine erhaltene Verbindung der Erfindung in eine andere Verbindung der Erfindung umwandelt, und/oder, falls gewünscht, dass man eine erhaltene freie Verbindung in ein Salz oder ein erhaltenes Salz in eine freie Verbindung oder in ein anderes Salz umwandelt, und/oder, falls gewünscht, dass man eine erhaltene Mischung von Isomeren oder Racematen in die einzelnen Isomere oder Racemate auftrennt, und/oder, falls gewünscht, dass man ein erhaltenes Racemat in die optischen Antipoden auftrennt.

**Revendications**

1. Composés de formule I

$$Y{-}\underset{R}{CH}{-}CH_2{-}\underset{R_0}{CH}{-}CONH{-}A{-}X \qquad (I)$$

dans laquelle X et Y représentent indépendamment un hydroxyméthyle ; un cyano ; un carboxy ; un carboxy modifié de façon fonctionnelle choisi parmi un carboxy estérifié, un carbamoyle, et un carbamoyle N-substitué ; un 5-tétrazolyle ; un 2-oxazolyle, un 4,5-dihydro-2-oxazolyle, un 2-imidazolyle ou un 4,5-dihydro-2-imidazolyle ou n'importe lequel de ces groupes substitué par un alcoyle inférieur ; R et $R_0$ représentent indépendamment un alcoyle inférieur, un ($C_3$-$C_7$)-cycloalcoyl-alcoyle inférieur, ou un aryl-alcoyle inférieur dans lequel l'aryle représente un phényle, un pyridyle, un thiényle, un furyle, un biphénylyle ou un naphtyle, chacun étant non substitué ou mono- ou di-substitué par un halogène, un alcoyle inférieur, un hydroxy, un acyloxy, un alcoxy inférieur, un trifluorométhyle ou un cyano ; A représente un ($C_2$-$C_5$)-alcoylène à chaîne droite ; ou A représente un ($C_2$-$C_5$)-alcoylène à chaîne droite substitué par un alcoyle inférieur, par un alcoylthio inférieur-alcoyle inférieur, par un hydroxy-alcoyle inférieur, par un acyloxy-alcoyle inférieur, par un alcoxy inférieur-alcoyle inférieur, par un amino ou un acylamino, par un amino-alcoyle inférieur, par un acylamino-alcoyle inférieur, par un ($C_3$-$C_7$)-cycloalcoyle, par un ($C_3$-$C_7$)-cycloalcoyl-alcoyle inférieur, par un aryle ou un aryl-alcoyle inférieur dans lequel l'aryle représente un phényle ou un phényle mono- ou disubstitué par un halogène, un alcoyle inférieur, un alcoxy inférieur, un hydroxy, un acyloxy, un trifluorométhyle ou un cyano ; ou A représente un phénylène ou un cyclohexylène ; ou des dérivés, pour médicaments acceptables en pharmacie, de n'importe lesquels de ces composés ayant un groupe carboxy libre ; ou des sels acceptables en pharmacie de n'importe lesquels de ces composés avec un groupe formant un sel.

2. Composés de formule II selon la revendication 1

$$HOOC{-}\underset{R_1}{CH}{-}CH_2{-}\underset{R_2}{CH}{-}CONH{-}A{-}COOH \qquad (II)$$

dans laquelle $R_1$ et $R_2$ représentent indépendamment un alcoyle inférieur, un ($C_3$-$C_7$)-cycloalcoyle-alcoyle inférieur, ou un aryl-alcoyle inférieur dans lequel l'aryle représente un phényle, un pyridyle, un thiényle, un furyle, un biphénylyle ou un naphtyle chacun étant non substitué ou mono- ou di-substitué par un halogène, un alcoyle inférieur, un hydroxy, un alcoxy inférieur, un trifluorométhyle ou un cyano ; A représente un ($C_2$-$C_5$)-alcoylène à chaîne droite ; ou A représente un ($C_2$-$C_5$)-alcoylène à chaîne droite substitué par un alcoyle inférieur, par un alcoylthio inférieur-alcoyle inférieur, par un hydroxy-alcoyle inférieur, par un acyloxy-alcoyle inférieur, par un alcoxy inférieur-alcoyle inférieur, par un amino ou un acylamino, par un amino-alcoyle inférieur, par un acylamino-alcoyle inférieur, par un ($C_3$-$C_7$)-cycloalcoyle, par un ($C_3$-$C_7$)-cycloalcoyl-alcoyle inférieur, par un aryle ou un aryl-alcoyle inférieur dans lequel l'aryle représente un phényle ou un phényle mono- ou disubstitué par un halogène, un alcoyle inférieur, un hydroxy, un alcoxy inférieur, un trifluorométhyle ou un cyano ; ou A représente un phénylène ou un cyclohexylène ; un dérivé d'acide mono- ou bis-carboxylique de ceux-ci dans lesquels le dérivé est choisi parmi un amide non substitué ou un mono- ou di-($C_1$-$C_{20}$)-alkylamide ; un alcoylène tertiaire inférieur, un oxyalcoylène ou un azaalcoylène amide dans lequel le groupe de l'alcoylène inférieur, l'oxyalcoylène ou l'azaalcoylène forme avec l'azote de l'amide un cycle à 5, 6 ou 7 membres, ou ledit alcoylène inférieur, oxalcoylène ou azaalcoylène amide est substitué sur le cycle par un alcoyle inférieur, un hydroxy-alcoyle inférieur ou par un alcanoyloxy inférieur-alcoyle inférieur ; un α-alcoxycarbonyl inférieur- ou α-carboxy-substitué alcoylamide inférieur ; un α-alcoxycarbonyl inférieur- ou α-carboxy-substitué aryl-alcoylamide inférieur dans lequel l'aryle représente, au choix, un phényle substitué comme défini ci-dessus ou le 3-indolyle ; un (amino ou acylamino)-alcoylamide inférieur ; un ($C_1$-$C_{20}$)-alcoyl ester ; un (amino, acylamino, mono- ou di-alcoylamino inférieur, un carboxy ou un alcoxy inférieur-carbonyl)-substitué alcoyl

inférieur ester ; un ester aryl-alcoyl inférieur dans lequel l'aryle représente au choix un phényle substitué comme défini ci-dessus ou le pyridyle ; un ester d'un alcanoyloxy inférieur-alcoyle inférieur ; un ester phtalidyle ; un (hydroxy, alcanoyloxy inférieur, ou un alcoxy inférieur)-substitué alcoxyméthyl inférieur ester ; un ester bicycloalcoyloxycarbonyl-alcoyl inférieur ayant jusqu'à 10 atomes de carbone dans le groupe bicycloalcoyle ; un hydroxyamide ; un alcoylsulfonylamide inférieur ; ou un ester 1-(alcoxy inférieur-carbonyloxy)-alcoyle inférieur ; un ester 3-cholestanyl ou 3-cholestény1 ; et aussi un ester monosaccharidyle ou un ester monosaccharidyle bloqué qui est un ester incorporant comme portion alcool un monosaccharide ou un monosaccharide bloqué, par exemple un aldopentose libre ou bloqué, un aldohexose, un cétopentose ou un cétohexose, sous forme d'une chaîne droite ou cyclique, par exemple la forme furanose ou pyranose, ou un acide libre ou bloqué glyconique ayant 5 ou 6 atomes de carbone ou une lactone correspondante ; un ester polyhydroxy-alcoyle inférieur ou polyhydroxy bloqué-alcoyle inférieur qui est un ester comportant comme portion alcool un polyhydroxy-alcane inférieur ou un polyhydroxy bloqué-alcane inférieur, par exemple un glycérol libre ou bloqué ou un érythritol libre ou bloqué ; et des sels acceptables en pharmacie de n'importe lesquels de ces composés avec un groupe formant un sel.

3. Composés de formule III selon la revendication 1

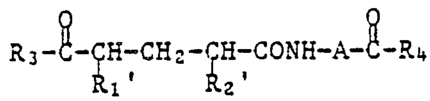

(III)

dans laquelle $R_1'$ et $R_2'$ représentent indépendamment un alcoyle inférieur ou un aryl-($C_1$-$C_4$)-alcoyle dans lequel l'aryle représente un phényle ou un phényle mono- ou di-substitué par un halogène, un alcoyle inférieur, un hydroxy, un alcoxy inférieur, un trifluorométhyle ou un cyano ; A représente un alcoylène ($C_2$-$C_5$) à chaîne droite ; ou A représente —($C_2$-$C_5$)-alcoylène à chaîne droite mono-substitué par un alcoyle inférieur, par un phényle ou un phényl-alcoyle inférieur, par un (halogène, alcoyl inférieur, hydroxy, trifluorométhyl ou alcoxy inférieur)-mono- ou di-substitué phényle ou phényl-alcoyle inférieur, par un alcoyl-thio-alcoyle inférieur, par un hydroxy-alcoyle inférieur, par un alcoxy inférieur-alcoyle inférieur, par un amino-alcoyle inférieur ou par un acylamino-alcoyle inférieur ; ou A représente un phénylène ou un cyclohexylène ; dans lequel $R_3$ et $R_4$ représentent chacun un hydroxy, un ($C_1$-$C_{20}$)-alcoxy ; un (amino, acylamino, mono- ou di-alcoylamino inférieur)-alcoxy inférieur ; un carboxy-alcoxy inférieur ; un alcoxycarbonyl inférieur-alcoxy inférieur ; un aryl-alcoxy inférieur dans lequel l'aryle représente, au choix, un (halogène, alcoyl inférieur, hydroxy ou alcoxy inférieur)-mono- ou di-substitué phényle ou pyridyle ; un (hydroxy, alcanoyloxy inférieur ou alcoxy inférieur)-alcoxy inférieur ; un (hydroxy, alcanoyloxy inférieur ou alcoxy inférieur)-alcoxyméthoxy inférieur ; un bicyclo-[2,2,1]-heptyloxycarbonyl-alcoxy inférieur ; un cholestan-3-oxy ou un cholest-5-en-oxy ; un 3-phtalidoxy ; en outre, un (alcoyl inférieur, alcoxy inférieur, halogène)- substitué 3-phtalidoxy, aussi un monosaccharidyloxy ou un monosaccharidyloxy bloqué représentant par exemple un glucosyloxy, un galactosyloxy, un mannosyloxy, un sorbosyloxy, un allosyloxy, un ribosyloxy, un arabinosyloxy, un ribonyloxy, un glyconyloxy, ou des formes cycliques correspondantes, par exemple des formes appropriées pyranose, furanose ou lactone, dans lesquelles les groupes hydroxy sont libres ou un ou plus, de façon appropriée, sont bloqués sous forme d'esters, par exemple un ester alcanoyl inférieur ou benzoyl, sous forme d'éthers, par exemple un éther alcoyl inférieur ou un éther benzyl, ou, lorsque deux groupes hydroxy voisins sont concernés, sous forme d'acétals ou de cétals, par exemple un dérivé d'un alcoylidène inférieur, d'un benzylidène ou d'un cycloalcoylidène à 5 ou 6 membres ; et aussi un polyhydroxy-alcoxy inférieur ou un polyhydroxy-alcoxy inférieur bloqué représentant par exemple un dihydroxypropyloxy ou un trihydroxybutyloxy dans lesquels les groupes hydroxy sont libres ou un ou plus, de façon appropriée, sont bloqués sous forme d'esters, par exemple d'un ester alcanoyl inférieur ou benzoyl, sous forme d'éthers, par exemple un éther alcoyl inférieur ou benzyl, ou, lorsque deux groupes hydroxy voisins sont concernés, sous forme d'acétals ou de cétals, par exemple un dérivé d'un alcoylidène inférieur, d'un benzylidène ou d'un cycloalcoylidène à 5 ou 6 membres ; et aussi un 1-(alcoxycarbonyloxy inférieur)-alcoxy inférieur ; un amino ; un mono- ou di-($C_1$-$C_{20}$)-alcoylamino ; un morpholino ; un N-alcoylpipérazino inférieur ; un pyrrolidino ; un pipéridino ; un perhydroazépino ; un (amino ou acylamino)-alcoylamino inférieur ; un α-(carboxy ou alcoxycarbonyl inférieur)-alcoylamino inférieur ; un arylalcoylamino inférieur dans lequel l'aryle est un phényle ou un 3-indolyle et qui peut être substitué sur le carbone en α par un carboxy ou un alcoxycarbonyle inférieur ; un hydroxyamino ; ou un alcoylsulfonylamino inférieur ; et des sels acceptables en pharmacie de n'importe lesquels de ces composés avec un groupe formant un sel.

4. Composés de formule III selon la revendication 3 dans laquelle $R_1'$ et $R_2'$ représentent indépendamment un aryl-($C_1$-$C_4$)-alcoyle dans lequel l'aryle représente un phényle ou un phényle mono- ou di-substitué par un halogène, un alcoyle inférieur, un hydroxy, un alcoxy inférieur, un trifluorométhyle ou un cyano ; A représente un ($C_2$-$C_5$)-alcoylène à chaîne droite, un ($C_2$-$C_5$)-alcoylène à chaîne droite substitué par un alcoyle inférieur, un phényle, un halophényle, ou substitué par un phényl-alcoyle inférieur ; ou A représente un phénylène ou un cyclohexylène ; $R_3$ et $R_4$ représentent indépendamment un hydroxy ; un ($C_1$-$C_{20}$)-alcoxy ; un (amino, mono- ou di-alcoylamino inférieur)-alcoxy inférieur ; un α-carboxy-alcoxy inférieur ; un α-alcoxycarbonyl inférieur-alcoxy inférieur ; un aryl-méthoxy dans lequel

l'aryle représente un phényle, un pyridyle, ou un (halogène, alcoyl inférieur ou alcoxy inférieur)-monosubstitué phényle ou pyridyle ; un (alcanoyloxy inférieur ou alcoxy inférieur)-méthoxy ; un (hydroxy, alcanoyloxy inférieur ou alcoxy inférieur)-éthoxyméthoxy ; un bicyclo[2,2,1]heptyl-oxycarbonylméthoxy ; un 1-(alcoxy-carbonyloxy inférieur)-alcoxy inférieur ; de plus, un 3-phtalidoxy ou un (alcoyl inférieur, alcoxy inférieur, halogène)-substitué 3-phtalidoxy, un cholestan-3-oxy ou un cholest-5-en-3-oxy ; aussi un monosaccharidyloxy ou un monosaccharidyloxy bloqué choisi parmi un glucosyloxy, un galactosyloxy, un mannosyloxy, un sorbosyloxy, un allosyloxy, un ribosyloxy, un arabinosyloxy, un ribonyloxy, un gluconyloxy, ou des formes cycliques correspondantes, par exemple des formes appropriées pyranose, furanose ou lactone, dans lesquelles les groupes hydroxy sont libres ou un ou plus, de façon appropriée, sont bloqués sous forme d'un ester alcanoyl inférieur ou benzoyl, sous forme d'un éther alcoyle inférieur ou benzyl, ou, lorsque deux groupes hydroxy voisins sont concernés, sous forme d'un dérivé d'alcoylidène inférieur, d'un benzylidène ou d'un cycloalcoylidène à 5 ou 6 membres ; un polyhydroxy-alcoxy inférieur ou un polyhydroxy-alcoxy inférieur bloqué choisi parmi un dihydroxypropyloxy ou trihydroxybutyloxy dans lesquels les groupes hydroxy sont libres ou un ou plus, de façon appropriée, sont bloqués sous forme d'un ester alcanoyl inférieur ou benzoyl, sous forme d'un éther alcoyl inférieur ou benzyl, ou, lorsque deux groupes hydroxy voisins sont concernés, sous forme d'un dérivé d'alcoylidène inférieur, de benzylidène ou d'un cycloalcoylidène à 5 ou 6 membres ; et aussi un amino ; un mono- ou di-$(C_1-C_{20})$-alcoylamino ; un morpholino ; un N-méthylpipérazino ; un pipéridino ; un perhydroazépino ; un amino-alcoylamino inférieur ; un α-(carboxy ou alcoxycarbonyl inférieur)-alcoylamino inférieur, un phényl-α-(carboxy ou alcoxycarbonyl inférieur)-alcoylamino inférieur ; un hydroxyamino ; ou un alcoylsulfonylamino inférieur ; et des sels acceptables en pharmacie de n'importe lesquels de ces composés avec un groupe basique ou acide formant un sel.

5. Composés de formule III selon la revendication 3 dans laquelle $R_3$ et $R_4$ représentent indépendamment un hydroxy, un $(C_1-C_{20})$-alcoxy, un pivaloyloxyuméthoxy, un bornyloxycarbonylméthoxy, un benzyloxy, un pyridylméthoxy, un α-carboxyéthoxy, un α-alcoxycarbonyléthoxy inférieur, un 3-phtalidoxy, en outre un monosaccharidyloxy ou un monosaccharidyloxy bloqué choisi parmi un glucofuranosyloxy, glucopyranosyloxy, galactopyranosyloxy, allofuranosyloxy, mannofuranosyloxy, ribofuranosyloxy, sorbofuranosyloxy, arabinofuranosyloxy et ribono-(1,4-lactone)-yloxy, dans lesquels les groupes hydroxy sont libres ou les groupes hydroxy sont bloqués sous forme d'un ester alcanoyl inférieur, sous forme d'un éther benzyl ou sous forme d'un dérivé d'isopropylidène, de benzylidène ou de cyclohexylidène ; et aussi d'un amino, d'un mono- ou d'un di-alcoylamino inférieur, d'un morpholino, d'un α-(carboxy ou alcoxycarbonyl inférieur)-alcoylamino inférieur, ou d'un alcoylsulfonylamino inférieur ; et des sels acceptables en pharmacie de n'importe lesquels de ces composés avec un groupe basique ou acide formant un sel.

6. Composés de formule III selon la revendication 3 dans laquelle les groupes $COR_3$ et $COR_4$ représentent indépendamment un carboxy ou un carboxy estérifié sous forme d'un ester acceptable en pharmacie dans lequel $R_3$ et $R_4$ représentent indépendamment un hydroxy, un alcoxy inférieur, un benzyloxy, un pyridylméthoxy, un pivaloyloxyméthoxy, un 3-phtalidoxy, aussi un monosaccharidyloxy ou un monosaccharidyloxy bloqué.

7. Composés de formule III selon la revendication 3 dans laquelle un des $R_3$ et $R_4$ représente un hydroxy et l'autre des $R_3$ et $R_4$ représente un monosaccharidyloxy bloqué.

8. Composés de formule III selon la revendication 3 dans laquelle un des $R_3$ et $R_4$ représente un hydroxy et l'autre des $R_3$ et $R_4$ représente un 3-phtalidoxy.

9. Composés de formule IV selon la revendication 1

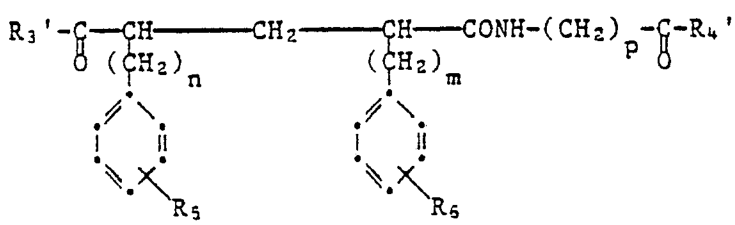

(IV)

dans laquelle m et n représentent indépendamment un nombre entier de 1 à 4 ; p représente un nombre entier de 2 à 4 ; $R_3'$ et $R_4'$ représentent un hydroxy ; $R_5$ et $R_6$ représentent indépendamment l'hydrogène, un halogène, un hydroxy, un alcoxy inférieur, un alcoyle inférieur ou un trifluorométhyle ; ou un dérivé correspondant pour médicament acceptable en pharmacie mono- ou di-(ester ou amide) ; et des sels acceptables en pharmacie de n'importe lesquels de ces composés avec un groupe carboxy libre ou un groupe basique formant un sel.

10. Composés de formule IV selon la revendication 9 dans laquelle m et n représentent indépendamment un nombre entier de 1 à 4 ; p représente un nombre entier de 2 à 4 ; $R_3'$ et $R_4'$ représentent indépendamment un hydroxy, un $(C_1-C_{20})$-alcoxy, un amino, un pivaloyloxyméthoxy, un bornyloxycarbonylméthoxy, un benzyloxy, un pyridylméthoxy, un 1-(alcoxycarbonyloxy inférieur)-alcoxy inférieur ou un alcoyl inférieur-sulfonylamino, en outre un 3-phtalidoxy ; et aussi un monosaccharidyloxy ou un

monosaccharidyloxy bloqué choisi parmi un glucofuranosyloxy, un glucopyranosyloxy, un galactopyranosyloxy, un allofuranosyloxy, un mannofuranosyloxy, un ribofuranosyloxy, un sorbofuranosyloxy, un arabinofuranosyloxy et un ribono(1,4-lactone)-yloxy, dans lesquels les groupes hydroxy sont libres ou les groupes hydroxy sont bloqués sous forme d'un ester alcanoyl inférieur, sous forme d'un éther benzyl ou sous forme d'un dérivé inférieur d'isopropylidène, de benzylidène ou de cyclohexylidène ; $R_5$ et $R_6$ représentent indépendamment un hydrogène, un halogène, un hydroxy, un alcoxy inférieur, un alcoyle inférieur ou un trifluorométhyle ; et des sels acceptables en pharmacie de n'importe lesquels de ces composés avec un groupe carboxy libre ou un groupe basique formant un sel.

11. Composés racémiques thréo de formule I selon la revendication 1.

12. Composés de formule I selon la revendication 1, ces composés étant présents sous la forme énantiomère décrite dans la formule Ia

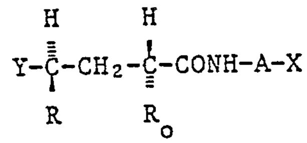

(Ia)

13. Composés de formule IV selon la revendication 9, ces composés étant présents sous la forme énantiomère décrite dans la formule IVa

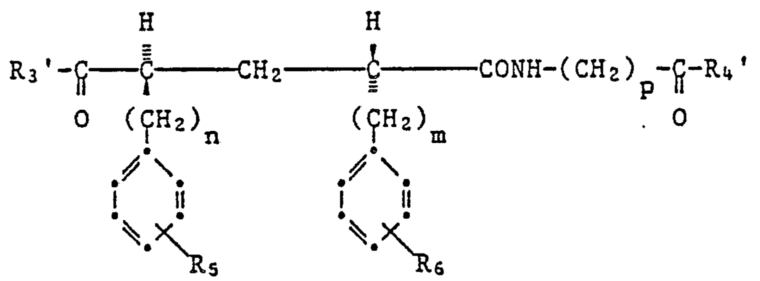

(IVa)

14. Composé selon la revendication 11, ce composé étant l'acide N-[(R*,R*)-2,4-dibenzyl-4-carboxybutyryl]-3-aminopropionique ou l'acide N-[(R*,R*)-2,4-dibenzyl-4-carboxybutyryl]-4-aminobutyrique ; un dérivé de ces composés mono- ou diester pour médicaments acceptable en pharmacie ; l'antipode S,S d'un tel composé ; ou un sel acceptable en pharmacie d'un tel composé ayant un groupe acide ou basique formant un sel.

15. Composé selon la revendication 9, ce composé étant l'acide N-[4-éthoxycarbonyl-2,4-dibenzylbutyryl]-3-aminopropionique, un stéréoisomère de celui-ci, un mélange de divers stéréoisomères de celui-ci ; ou un sel de celui-ci acceptable en pharmacie.

16. Composé selon la revendication 9, ce composé étant l'acide N-[4-éthoxycarbonyl-2,4-dibenzylbutyryl]-4-aminobutyrique, un stéréoisomère de celui-ci, un mélange de différents stéréoisomères de celui-ci ; ou un sel de celui-ci acceptable en pharmacie.

17. Composé selon la revendication 9, ce composé étant l'acide N-(2,4-dibenzyl-4-carboxybutyryl)-4-aminobutyrique ou l'acide N-(2,4-dibenzyl-4-carboxybutyryl)-3-aminopropionique, un stéréoisomère de celui-ci, des mélanges de ces stéréoisomères et des sels de ceux-ci acceptables en pharmacie.

18. Préparations pharmaceutiques contenant un composé de formule I selon la revendication 1 ou un sel de celui-ci acceptable en pharmacie.

19. Composé de formule I selon la revendication 1 et sels de celui-ci acceptables en pharmacie pour emploi dans une méthode pour le traitement prophylactique et/ou thérapeutique du corps animal ou humain.

20. Composé de formule I selon la revendication 1 et sels de celui-ci acceptables en pharmacie comme composés ayant une activité analgésique.

21. Composé de formule I selon la revendication 1 et sels de celui-ci acceptables en pharmacie comme composés ayant une activité antihypertensive.

22. Emploi de composés de formule I selon la revendication 1 ou de sels de ceux-ci acceptables en pharmacie pour la fabrication de préparations pharmaceutiques.

23. Procédé pour la fabrication de composés de formule I selon la revendication 1, ou de sels de ceux-ci, comprenant : la condensation d'un composé de formule V

$$NH_2—A—X \qquad (V)$$

dans laquelle A et X ont les significations données sous la formule I, sous forme bloquée temporairement si nécessaire, avec un composé de formule VI

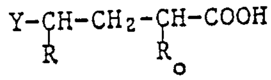 (VI)

ou un dérivé fonctionnel réactif de celui-ci, dans laquelle R, $R_0$ et Y ont les significations définies sous la formule I, sous forme bloquée temporairement si nécessaire ; et, si on bloque temporairement un ou plusieurs groupes réactifs gênants quelconques, élimination du ou de ces groupes de blocage ; et, si on le désire, condensation d'un diacide de formule I obtenu, dans lequel X et Y sont un carboxy, ou un dérivé monoester de celui-ci, avec un agent estérifiant de formule VII

$$R_7\text{—}Z \qquad \text{(VII)}$$

dans laquelle Z représente un hydroxy ou un groupe hydroxyle estérifié réactif ; et $R_7$ représente un quelconque des radicaux ester définis pour la formule I de façon à obtenir un dérivé mono- ou bis-fonctionnel dudit diacide de formule I dans lequel soit l'un soit les deux des groupes carboxy sont estérifiés par des radicaux identiques ou différents, et, si on le désire, transformation d'un composé de l'invention résultant en un autre composé de l'invention, et/ou, si on le désire, transformation d'un composé libre résultant en un sel ou d'un sel résultant en composé libre ou en un autre sel, et/ou, si on le désire, séparation d'un mélange d'isomères ou de racémates obtenus en isomères ou racémates isolés, et/ou, si on le désire, résolution d'un racémate obtenu en antipodes optiques.